# EUROPÄISCHE PATENTSCHRIFT

(11) **EP 1 836 489 B1**
(45) Veröffentlichungstag und Bekanntmachung des Hinweises auf die Patenterteilung: **30.06.2010**
(21) Anmeldenummer: 05817936.7
(22) Anmeldetag: 08.12.2005
(51) Int. Cl.: G01N 33/50

(54) **VERFAHREN ZUM TESTEN VON SUBSTANZEN AN BIOMATRICES**
METHOD FOR TESTING SUBSTANCES ON BIOMATRICES
PROCEDE DE TEST DE SUBSTANCES SUR DES BIOMATRICES

(30) Priorität: 08.12.2004 DE 102004059169
(43) Veröffentlichungstag der Anmeldung: 26.09.2007
(73) Patentinhaber: Humanautocell GmbH, 80802 München (DE)
(72) Erfinder: GÖRNE, Martin, 22337 Hamburg (DE); KAUFMANN, Peter-Matthias, 30900 Wedemark (DE)
(74) Vertreter: Reitstötter - Kinzebach
(86) Internationale Anmeldenummer: PCT/EP2005/013178
(87) Internationale Veröffentlichungsnummer: WO 2006/061229

(56) Entgegenhaltungen:
- US-A1- 2002 094 514
- US-A1- 2004 063 206
- HOU Q ET AL: "Porous polymeric structures for tissue engineering prepared by a coagulation, compression moulding and salt leaching technique" BIOMATERIALS, ELSEVIER SCIENCE PUBLISHERS BV., BARKING, GB, Bd. 24, Nr. 11, Mai 2003 (2003-05), Seiten 1937-1947, XP004412414 ISSN: 0142-9612
- HARRIS L D ET AL: "OPEN PORE BIODEGRADABLE MATRICES FORMED WITH GAS FOAMING" JOURNAL OF BIOMEDICAL MATERIALS RESEARCH, WILEY, NEW YORK, NY, US, Bd. 42, 1998, Seiten 396-402, XP001021110 ISSN: 0021-9304
- KAUFMANN P M ET AL: "Is there an optimal concentration of cotransplanted islets of Langerhans for stimulation of hepatocytes in three dimensional matrices?" TRANSPLANTATION 27 JUL 1999 UNITED STATES, Bd. 68, Nr. 2, 27. Juli 1999 (1999-07-27), Seiten 272-279, XP002369920 ISSN: 0041-1337

## Beschreibung

Die vorliegende Erfindung betrifft ein Verfahren zum Testen von Substanzen an Biomatrices. Die Biomatrices stellen Gewebeäquivalente dar, d.h. Gewebszellen auf porösen Matrices, die auf biologisch verträglichen Polymeren oder Polymergemischen basieren. Ein weiterer Gegenstand der vorliegenden Erfindung ist daher die Verwendung der Gewebeäquivalente zum Testen von Substanzen. Ein Verfahren zur Herstellung poröser Matrices sowie die nach diesem Verfahren erhältlichen Matrices, ein spezielles Verfahren zur Gewinnung von Zellen für die Beimpfung der Matrices und die Herstellung der Gewebeäquivalente werden ebenfalls beschrieben.

Tissue engineering ist ein interdisziplinäres Gebiet, das Ingenieur- und Materialwissenschaften mit der Medizin verbindet. Ziel ist es, geschädigtes Gewebe wiederherzustellen oder seine Funktion zu verbessern.

Das Prinzip des Tissue engineering ist denkbar einfach: Zunächst werden Zellen bereitsgestellt, z.B. einem Organismus einige Zellen entnommen und *in vitro* vermehrt. Die vermehrten Zellen können dann in eine Gerüstsubstanz eingebettet werden, wodurch ein komplettes, lebendes Gewebeäquivalent entsteht.

Von besonderer Bedeutung für die Funktionsfähigkeit der Gewebeäquivalente sind Art und Aufbau der verwendeten Gerüstsubstanz, im folgenden auch Matrix genannt. Abgesehen von dem zu verwendenden Material, nämlich in der Regel biologisch abbaubaren Polymeren, spielen Porengröße, Porosität und Oberfläche genauso wie die Porengestalt, die Morphologie der Porenwand und die Konnektivität zwischen den Poren eine entscheidende Rolle für die weitere Entwicklung der in der Gerüstsubstanz eingebetteten Zellen und letztendlich für den dreidimensionalen Aufbau der Gewebeäquivalente. Dreidimensionale Matrices wurden beispielsweise hinsichtlich einer Verwendung für die Transplantation von Leberzellen untersucht (Kaufmann P. M. et al. (1999), Transplantation 68(2), S.272-279).

Verfahren zur Erzeugung derartiger Biomatrices sind bereits bekannt. So wurden bereits Techniken aus dem Textilbereich angewendet, um webartige und auch vliesartige, faserige Biomatrices herzustellen. Ein weiteres geläufiges Verfahren, bei dem Salzkristalle zunächst in das biologisch abbaubare Polymer eingearbeitet und anschließend wieder herausgelöst werden, ermöglicht es, die Porengröße über die Größe der Salzpartikel und die Porosität über das Salz/Polymerverhältnis zu kontrollieren (WO 98/44027; Hou Q. et a/. (2003), Biomaterials 24, S.1937-47; Harris L. D. et al. (1998), J. Biomed. Mater. Res. 42, S.396-402). Bei einer Abwandlung des Verfahrens werden die in einem Lösungsmittel gelösten, biologisch abbaubaren Polymere auf ein sogenanntes porogenes Material aufgetragen, das anschließend aus dem Verbundmaterial wieder herausgelöst wird, wodurch Poren mit der Gestalt des negativen Abbildes besagten porogenen Materials hinterlassen werden (WO 01/87575 A2). Auch beschichtete Matrices sowie Matrices auf Basis eines lyophilisierten Hydrogels sind bereits bekannt (siehe z.B. WO 99/09149 A1 und US2004/0063206 A1).

Die Prüfung, ob eine bestimmte Substanz für den menschlichen oder nichtmenschlichen, tierischen Körper toxisch ist, ist bei der Entwicklung eines Arzneimittels für den human- bzw. veterinärmedizinischen Bereich ein entscheidender Schritt. Grundsätzlich ist es wünschenswert, eine potenzielle Toxizität so früh wie möglich zu erkennen. Nicht selten mussten Wirkstoffe vom Markt genommen werden, weist sie zu einem akuten Leberversagen führten, also eine zunächst unerkannte Lebertoxizität aufwiesen.

In der Vergangenheit wurde die toxikologische Prüfung von Arzneimittelwirkstoffen, Pestiziden, Lebensmittelzusätzen und weiteren Umwelstoffen entweder *in vivo* in Versuchstieren oder an *in vitro*-Systemen, wie Bakterien (z.B. Ames-Test) und Tierzellkulturen, durchgeführt. In den bakteriellen Testsystemen und einigen der Tierzellkulturen fehlt allerdings jegliche metabolische Aktivität, weswegen toxische Stoffwechselprodukte mit diesen Systemen nicht erkannt werden können. Um diesem Problem Rechnung zu tragen, hat man in der Vergangenheit zwar bestimmte Enzymextrakte, beispielsweise Rattenleberextrakte, eingesetzt. Die auf diese Weise simulierte Stoffwechselaktivität stimmt aber nicht notwendigerweise mit der im Menschen oder Tieren überein. Außerdem kann es vorkommen, dass hochreaktive Metabolite ihre Zielmoleküle nicht erreichen und damit nicht nachgewiesen werden.

Auch hat es nicht an Versuchen gefehlt, differenzierte Humanzellen mit bestimmten Stoffwechselaktivitäten *in vitro* zu kultivieren und so ein Testsystem mit einer angemessenen Nähe zum humanen Stoffwechsel zur Verfügung zu stellen. Da differenzierte Zellen aber nur bedingt kultivierbar sind, unter anderem weil die bei der Zubereitung von Zellkulturen zwecks Vereinzelung der Zellen notwendigerweise durchzuführenden mechanischen und/oder enzymatischen Behandlungen zu einem Verlust und/oder einer Beschädigung der Zell-Zell-Kontakte führen, waren bestimmte Maßnahmen erforderlich, um überhaupt eine entsprechende Zellkultur etablieren zu können. So hat man beispielsweise versucht, Leberzellen zu immortalisieren, was aber zu Artefakten führen kann. Andererseits stirbt Gewebe in Kultur innerhalb kürzester Zeit normalerweise ab, da ein ausreichender Austausch von Nährstoffen und Stoffwechselprodukten in der Regel nicht gewährleistet ist.

So sind die bekannten *in vitro*-Systeme vor allem mit dem Nachteil behaftet, den komplexen Stoffwechsel eines menschlichen oder tierischen Organismus nicht nachstellen zu können. Damit sind die an diesen Systemen erhaltenen Ergebnisse noch weniger übertragbar als die Ergebnisse von Tierversuchen. Das Risiko einer Verabreichung der zu testenden Substanzen an bzw. Aufnahme insbesondere durch den Menschen lässt sich möglicherweise nicht oder nur unzureichend bestimmen.

Die der vorliegenden Erfindung zugrunde liegende Aufgabe, ein funktionsfähiges Gewebeäquivalent zur Verfügung zu stellen, mit dem sich besagte Tests an Substanzen in zufriedenstellender Weise durchführen lassen, löst die Erfindung durch bestimmte Biomatrices, die mit einem speziellen Verfahren erhältlich sind und zum Aufbau eines entsprechenden Gewebeäquivalents dienen.

Gegenstand der vorliegenden Erfindung ist daher das in den Patentansprüchen definierte Verfahren. Es handelt sich um ein *in vitro* Verfahren.

Bei den zu testenden Substanzen handelt es sich um Substanzen, mit denen Menschen oder nichtmenschliche Tiere, insbesondere Haus- oder Nutztiere, in Kontakt kommen oder kommen könnten, also insbesondere solche, die vom Menschen oder einem nichtmenschlichen Tier aufgenommen werden oder werden könnten. Somit gehören zu diesen Substanzen insbesondere Wirkstoffe aus den Bereichen Pharmazie und Pflanzenschutz, Nahrungsmittelzusätze sowie eine Fülle von Umweltstoffen, mit denen der Mensch oder das nichtmenschliche Tier in Kontakt kommen könnte.

Das erfindungsgemäße Verfahren dient prinzipiell dazu, Wechselwirkungen einer zu testenden Substanz mit dem Gewebeäquivalent zu untersuchen. Wechselwirkung meint in diesem Zusammenhang sowohl eine Wirkung der Substanz auf das Gewebeäquivalent als auch eine Wirkung des Gewebeäquivalents auf die Substanz.

So lässt sich mit dem erfindungsgemäßen Verfahren insbesondere feststellen, ob sich das Gewebeäquivalent unter dem Einwirken der Substanz verändert. Eine Feststellung dieser Art beinhaltet in der Regel die Untersuchung wenigstens eines Zustandes (Parameters) an dem Gewebeäquivalent oder an einem wenigstens 1 Zelle umfassenden Teil davon. Als Parameter sind grundsätzlich sämtliche einen bestimmten Zustand des Gewebeäquivalents beschreibende Beobachtungs- oder Messgrößen brauchbar. Hierzu gehören beispielsweise zytologische Parameter, wie die Zellmorphologie, Zellvitalität und Zellteilungsrate, biochemische Parameter, wie bestimmte Stoffwechselaktivitäten, z.B. die Induktion bestimmter Enzyme, und die Bildung bestimmter Stoffwechselprodukte, und molekularbiologische Parameter, wie das Vorliegen bestimmter Nukleinsäuren und Proteine.

Veränderungen der Zellmorphologie kann man visuell, beispielsweise unter dem Mikroskop, untersuchen. Insbesondere kann man die Zellgröße bestimmen.

Die Vitalität von Zellen des Gewebeäquivalents lässt sich in an sich bekannter Weise, beispielsweise mit Hilfe bekannter Färbemethoden, bestimmen. Die Verwendung von Trypan-Blau, Neutralrot oder weiterer chromogener Substrate wie 3-(4,5-Dimethylthiazol-2-yl)-5-(3-carboxymethoxyphenyl)-2-(4-sulfophenyl)-2H-tetrazolium (Lebendtotfärbungen) ist dem Fachmann zu diesem Zweck hinlänglich bekannt. Ferner sei die elektronische Vitalitätsbestimmung erwähnt, wie mittels Stromausschlussverfahren (CASY®-Technologie).

Bestimmte Enzymaktivitäten, beispielsweise die an Phase-I- und/oder Phase-II-Biotransformationen beteiligten Enzyme, lassen sich in gewohnter Weise biochemisch bestimmen, beispielsweise indem man Substrate verwendet, deren Umsetzung zu einem nachweisbaren und möglichst quantifizierbaren Produkt führt. Nukleinsäuren und Proteine, z.B. Enzyme, lassen sich mit den gängigen molekularbiologischen Analysemethoden bestimmen.

So lässt sich insbesondere eine Induktion einer Reihe von Cytochromen P450 (z.B. 1A1, 1A2, 2A1, 3A4, 3A5, 2B6, 2C8, 2C9, 2C11, 2C19, 2D6, 2E1) enzymatisch oder auch molekularbiologisch nachweisen.

Diesem Aspekt zufolge richtet sich das erfindungsgemäße Verfahren insbesondere auf das Testen von Substanzen, um zu bestimmen, ob die Testsubstanz einen toxischen Effekt auf Zellen des Gewebeäquivalents hat (Zytotoxizität). In diesem Sinne sind von den vorstehend genannten Parametern diejenigen bevorzugt, die einen zytotoxischen Effekt wiedergeben können.

In der Regel ist eine Veränderung des Gewebeäquivalents an den Zellen des Gewebeäquivalents festzustellen. In bestimmten Fällen kann eine Veränderung des Gewebeäquivalents aber auch indirekt anhand einer Veränderung des Kulturmediums festgestellt werden, z. B. wenn das Gewebeäquivalent unter dem Einwirken der Testsubstanz ein Stoffwechselprodukt bildet und dies in das umgebende Kulturmedium abgibt.

Andererseits lässt sich mit dem erfindungsgemäßen Verfahren auch feststellen, ob die Testsubstanz unter Einwirkung des Gewebeäquivalents eine Veränderung erfährt. Zu solchen Veränderungen gehören insbesondere eine Änderung der chemischen Struktur der Testsubstanz und die Bildung von Addukten der Testsubstanz mit weiteren Substanzen, die vom Gewebeäquivalent zur Verfügung gestellt werden. Die unter Einwirkung des Gewebeäquivalents erfolgende Veränderung der Testsubstanz soll hier als Biotransformation und die daraus resultierenden, von der Testsubstanz abstammenden Produkte als Metaboliten bezeichnet werden. Zu solchen Biotransformationen gehören insbesondere Phase-I-Biotransformationen, wie Hydroxylierungen, und Phase II-Biotransformationen, wie eine Glucoronidierung, Sulfatierung, Methylierung, Acetylierung, Aminosäurekonjugation und Glutathionkonjugation. Die Phase-I-Biotransformationen dienen in der Regel dazu, die Polarität der Testsubstanz zu erhöhen und/oder in diese chemisch reaktive Gruppen einzuführen, an denen anschließend die Phase-II-Transformationen stattfinden können.

Diesem Aspekt zufolge richtet sich das erfindungsgemäße Verfahren insbesondere auf das Testen von Substanzen, um zu bestimmen, ob die Testsubstanz von den Zellen des Gewebeäquivalents metabolisiert wird (Biotransformation).

Die an solchen Biotransformationen beteiligten Produkte, also insbesondere die zu testende Substanz als Edukt sowie die von ihr abstammenden Metaboliten, lassen sich in der Regel mit herkömmlichen Analysemethoden bestimmen, wobei das Kulturmedium und/oder die Zellen des Gewebeäquivalents der Bestimmung unterzogen werden kann. In diesem Zusammenhang sind insbesondere chromatographische Techniken wie die HPLC, spektrometrische Verfahren, wie die Massenspektrometrie, sowie immunologische Verfahren, mit denen die Metabolite mit Hilfe von geeigneten Antikörpern nachgewiesen werden, zu nennen.

Zur Durchführung des erfindungsgemäßen Verfahrens wird das Gewebeäquivalent in der Regel in wässrigem Medium gehalten, wodurch Nährstoffe zugeführt und Stoffwechselprodukte abgeführt werden, und der notwendige Gasaustausch gewährleistet ist. Dazu enthält das Medium in der Regel die erforderlichen Nährstoffe. In diesem Zusammenhang sei auf die einschlägigen Medien zur Kultivierung humaner Zellen verwiesen. Für Lebergewebeäquivalente, die im erfindungsgemäßen Verfahren bevorzugt eingesetzt werden, hat sich beispielsweise gewöhnliches Williams-Medium E (mit 10 % fötalem Kälberserum supplementiert) als geeignet erwiesen. Ferner sind Humanseren bzw. Fraktionen davon geeignet.

Um eine ausreichende Nährstoffzufuhr bzw. einen hinreichenden Gasaustausch zu gewährleisten, wird das Medium in der Regel bewegt. Geeignete Vorrichtungen, in denen erfindungsgemäße Gewebeäquivalente kultiviert werden können, sind dem Fachmann hinlänglich bekannt und finden sich insbesondere in dem Bereich der Bioreaktoren.

Der oder die Testsubstanzen werden dem Medium, das in der Regel bereits das Gewebeäquivalent enthält, zu einem geeigneten Zeitpunkt zugesetzt. Zweckmäßigerweise wird man verschiedene Konzentration testen wollen, weshalb man mit der geringsten Konzentration beginnt. Mehrere Testsubstanzen können dem Medium als Substanzgemisch oder getrennt zugesetzt werden.

Die Bestimmung, ob das Einwirken der Substanz oder der Substanzen zu einer Veränderung des Gewebeäquivalents und/oder der Substanz(en) geführt hat, beinhaltet in der Regel wenigstens zwei Bestimmungen eines Zustandes (Parameters), nämlich eine Bestimmung vor dem Einwirken und eine Bestimmung nach Einwirkung. Ergibt der Vergleich der mit beiden Bestimmungen erhaltenen Ergebnisse eine Abweichung, liegt eine Veränderung vor. Insbesondere kann man mehr als zwei Bestimmungen desselben Zustandes und/oder Bestimmungen mehrerer Zustände durchführen. Dabei ist es eine Sache der Zweckmäßigkeit, mehrere gleichartige Gewebeäquivalente in demselben Medium vorzusehen, so dass für jede Bestimmung ein Gewebeäquivalente entnommen werden kann, ohne die verbleibenden Gewebeäquivalente zu beeinflussen.

Im folgenden werden die Gewebeäquivalente selbst sowie die ihnen zugrundeliegenden Matrices und deren Herstellung beschrieben.

Der Porositätsgrad ist die zahlenmäßige Angabe in % zum Anteil des Porenvolumens am Gesamtvolumen der Matrix.

Mit Poren bezeichnet man die in der erfindungsgemäßen Matrix vorhandenen Hohlräume, die im vorliegenden Fall im 2-dimensionalen Schnitt eine eckige, insbesondere oktogonale bzw. 3-dimensional gesehen eine kantige Gestalt haben. Vorzugsweise ist die Gestalt des weiteren durch Ausziehungen gekennzeichnet, so dass man die Gestalt der Hohlräume mit der Form von Nervenzellen vergleichen kann. Die Größe einer Pore kann mit Hilfe eines Durchmessers angegeben werden, das heißt dem Mittel aus dem längsten und dem kürzesten Durchmesser der im 2-dimensionalen Schnitt erkennbaren Poren.

Eine erfindungsgemäße Matrix weist Poren mit unterschiedlichen Größen auf, wobei die Größen über einen bestimmten Bereich verteilt sind (Porengrößenverteilung, wie in den Ansprüchen definiert). Erfindungsgemäß von Bedeutung ist, dass eine Matrix eine breite Porengrößenverteilung aufweist, die breiter ist als der Bereich, der sich von Poren mit einer Größe im Bereich von etwa 150 µm bis zu Poren mit einer Größe im Bereich von etwa 300 µm erstreckt. Demnach weist eine erfindungsgemäße Matrix Poren mit einer Größe von 130 µm oder weniger auf. Außerdem weist eine erfindungsgemäße Matrix Poren mit einer Größe von 370 µm oder mehr auf. Diese Werte lassen sich beliebig zu Mindestbereichen, über die sich die Porengrößenverteilung erstreckt, kombinieren, wobei insbesondere der Bereich 130 bis 370 µm zu nennen ist. Insbesondere weist die jeweilige Porengrößenverteilung Häufigkeitsmaxima außerhalb des Bereichs von 150 bis 300 µm auf, d.h. ein Häufigkeitsmaximum liegt oberhalb einer Porengröße von 300 µm und ein weiteres Häufigkeitsmaximum unterhalb einer Porengrößen von 150 µm.

Eine typische erfindungsgemäße Matrix weist folgende Porengrößenverteilung auf. Etwa 0,5 % bis 6 %, vorzugsweise etwa 1 % bis 5 %, noch bevorzugter etwa 2 % bis 4 % und insbesondere etwa 3 % Poren mit einem mittleren Durchmesser im Bereich von 70 bis 100 µm; etwa 2 % bis 8 %, vorzugsweise etwa 3 % bis 7 %, noch bevorzugter etwa 4 % bis 6 % und insbesondere etwa 5 % Poren mit einem mittleren Durchmesser im Bereich von 101 bis 115 µm; etwa 2 % bis 8 %, vorzugsweise etwa 3 % bis 7 %, noch bevorzugter etwa 4 % bis 6 % und insbesondere etwa 5 % Poren mit einem mittleren Durchmesser im Bereich von 116 bis 130 µm; etwa 1 % bis 7 %, vorzugsweise etwa 2 % bis 6 %, noch bevorzugter etwa 3 % bis 5 % und insbesondere etwa 4 % Poren mit einem mittleren Durchmesser im Bereich von 131 bis 300 µm; etwa 11 % bis 23 %, vorzugsweise etwa 13 % bis 21 %, noch bevorzugter etwa 15 % bis 19 % und insbesondere etwa 17 % Poren mit einem mittleren Durchmesser im Bereich von 301 bis 330 µm; etwa 4 % bis 10 %, vorzugsweise etwa 5 % bis 9 %, noch bevorzugter etwa 6 % bis 8 % und insbesondere etwa 7 % Poren mit einem mittleren Durchmesser im Bereich von 331 bis 360 µm; etwa 5 % bis 17 %, vorzugsweise etwa 7 % bis 15 %, noch bevorzugter etwa 9 % bis 13 % und insbesondere etwa 11 % Poren mit einem mittleren Durchmesser im Bereich von 361 bis 390 µm; etwa 7 % bis 19 %, vorzugsweise etwa 9 % bis 17 %, noch bevorzugter etwa 11 % bis 15 % und insbesondere etwa 13 % Poren mit einem mittleren Durchmesser im Bereich von 391 bis 420 µm; etwa 3 % bis 9 %, vorzugsweise etwa 4 % bis 8 %, noch bevorzugter etwa 5 % bis 7 % und insbesondere etwa 6 % Poren mit einem mittleren Durchmesser im Bereich von 421 bis 450 µm; etwa 12 % bis 24 %, vorzugsweise etwa 14 % bis 22 %, noch bevorzugter etwa 16 % bis 20 % und insbesondere etwa 18 % Poren mit einem mittleren Durchmesser im Bereich von 451 bis 480 µm; und etwa 5 % bis 17 %, vorzugsweise etwa 7 % bis 15 %, noch bevorzugter etwa 9 % bis 13 % und insbesondere etwa 11 % Poren mit einem mittleren Durchmesser im Bereich von 481 bis 510 µm. Es ergibt sich also in der Regel eine Porengrößenverteilung mit mehr als einem Maximum, was einer Häufung von Poren in mehr als einem Größenbereich entspricht. Dies ist für die Eigenschaften erfindungsgemäßer Matrices von besonderer Bedeutung.

Das Hohlraumvolumen und damit der Porositätsgrad sind in an sich bekannter Weise durch Porosimetrie zu bestimmen.

Die Porengrößen und damit auch die Porengrößenverteilung können beispielsweise durch Rasterelektronenmikroskopie bestimmt werden. Dazu werden dünne Schnitte der zu untersuchenden Matrix angefertigt und mit Gold überzogen. Die rasterelektronenmikroskopischen Aufnahmen werden ausgewertet, indem man sämtliche Poren einer definierten Fläche ausmisst, d.h. für jede Pore den längsten und den kürzesten Durchmesser bestimmt, aus beiden Werten die Summe bildet und die Summe durch 2 dividiert.

Der Begriff "Matrix" bezieht sich auf einen dreidimensionalen Träger, der für die Ansiedlung von Zellen geeignet ist. In diesem Sinne dient die Matrix als dreidimensionale Strukturvorlage (Templat) für die Ansiedlung von Zellen bzw. Geweben. Diese Ansiedlung erfolgt vorzugsweise in vitro.

Das Polymer kann im Prinzip jedes im Fachgebiet verwendbare Polymer sein. Hierzu gehören insbesondere biologisch verträgliche Polymere, die eine Ansiedlung von lebenden Zellen auf dem Polymer erlauben. Es können Polymere zum Einsatz kommen, die im Wesentlichen biologisch nicht abbaubar oder die zumindest zum überwiegenden Teil biologisch abbaubar sind.

Der Ausdruck "biologisch abbaubar" bezieht sich auf ein Material, das Lebewesen (oder von Lebewesen ableitbare Körperflüssigkeiten oder Zellkulturen) in verstoffwechselbare Produkte zu überführen vermögen. Zu biologisch abbaubaren Polymeren gehören beispielsweise bioresorbierbare und/oder bioerodierbare Polymere. Bioerodierbar bezeichnet die Fähigkeit, in biologischen Flüssigkeiten lösbar oder suspendierbar zu sein. Bioresorbierbar meint die Fähigkeit, von Zellen, Gewebe oder Flüssigkeiten eines Lebewesens aufgenommen werden zu können.

Zur erfindungsgemäß geeigneten biologisch abbaubaren Polymeren gehören im Prinzip sämtliche im Fachbereich verwendbaren Polymere, wozu neben den im Bereich des Tissue engineering bereits etablierten Polymeren beispielsweise auch Polymere gehören, die in Wirkstoffabgabevorrichtungen, wie Pflastern und Wirkstoffimplantaten, Eingang gefunden haben.

Zu geeigneten natürlichen Polymeren gehören beispielsweise Polypeptide wie Albumin, Fibrinogen, Collagen und Gelatine, sowie Polysaccharide, wie Chitin, Chitosan, Alginat und Agarose. Unter Umständen können diese natürlichen Polymere auch modifiziert sein, beispielsweise können Proteine wie Collagen quervernetzt sein.

Zu geeigneten synthetischen Polymeren gehören beispielsweise bestimmte Polyanhydride, insbesondere Poly(sebacinsäure-hexadecandisäure), Poly(s-caprolacton), Poly(orthoester), und vor allem Poly(α-hydroxyester) wie Poly(glykolsäure), Poly(milchsäure) und Poly(glykolsäuremilchsäure). So basieren die erfindungsgemäßen Matrices und Implantate vorzugsweise auf biologisch abbaubaren Polymeren, die Wiederholungseinheiten der Formel (I) enthalten: 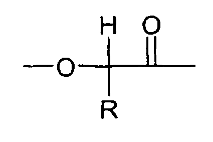 worin R¹ für Wasserstoff oder Methyl steht. Was die Milchsäureeinheiten angeht, so wird die L-Form (das S-Enantiomer) bevorzugt. Als besonders bevorzugtes Polymer ist Poly(glykolsäuremilchsäure) mit einem Glykolsäure zu Milchsäure-Verhältnis von 99:1 bis 1:99, vorzugsweise 10:90 bis 90:10, beispielsweise 15:85 mol% zu nennen.

Gemische aus zwei oder mehreren Polymeren können ebenfalls zweckmäßig sein.

Neben der Art des Polymers bestimmt auch sein Molekulargewicht die Eigenschaften der resultierenden Matrix mit. Allgemein gilt, dass die Porosität der Matrix mit zunehmendem Molekulargewicht des verwendeten Polymers abnimmt. Dies gilt insbesondere dann, wenn bei der Herstellung der Matrix das Material geschäumt wird, d.h. unter Druck mit einem Gas wie CO₂ versetzt wird, das sich zunächst in dem Polymer löst und bei Absenkung des Drucks Poren bildet.

Ferner wirkt sich die Kristallinität des eingesetzten Polymers auf die Eigenschaften der resultierenden Matrix aus. Hier gilt, dass die Porosität der resultierenden Matrix mit abnehmender Kristallinität im allgemeinen zunimmt, weshalb amorphes Polymer insbesondere für Matrices mit hoher Porosität bevorzugt ist. Auch dieser Aspekt spielt insbesondere dann eine Rolle; wenn das Material bei der Herstellung der Matrix geschäumt wird.

Die porösen Matrices auf Basis eines biologisch verträglichen Polymers können des Weiteren dadurch gekennzeichnet sein, dass die Oberfläche der Matrix mit wenigstens einem extrazellulären Matrixprotein beschichtet ist.

Extrazelluläre Matrixproteine sind allgemein bekannt. Erfindungsgemäß bevorzugt sind Collagene, insbesonders Collagene des Typs I und IV, Laminin und Fibronectin. Diese Proteine können in an sich bekannter Weise in aufgereinigter Form hergestellt oder auch kommerziell erworben werden. Gemäß einer Ausführungsform enthalten Beschichtungen erfindungsgemäßer Matrices als extrazelluläres Matrixprotein Fibronectin. Gemäß einer weiteren Ausführungsform enthalten Beschichtungen erfindungsgemäßer Matrices als extrazelluläres Matrixprotein ein Gemisch aus Collagen vom Typ I, Laminin und Collagen vom Typ IV, wobei es in diesem Fall bevorzugt ist, dass das Gemisch die Proteine in etwa gleichen Gewichtsanteilen enthält.

Erfindungsgemäß besonders bevorzugt sind Matrices, die in der oben beschriebenen Art und Weise beschichtet sind und die wenigstens eines der folgenden zusätzlichen Kriterien erfüllen:
- Die Poren der Matrices weisen die oben angegebenen Porengrößen bzw. Porengrößenverteilung auf;
- der Porositätsgrad beträgt 93 bis 98 %;
- die Poren weisen die oben angegebene Gestalt auf:

- das biologisch verträgliche Polymer ist eines der oben angegebenen natürlichen oder synthetischen Polymere, insbesondere Poly(glycolsäuremilchsäure) mit einem Milchsäureanteil von etwa 85 mol-% und einem Glykolsäureanteil von etwa 15 mol-%.

Derart beschichtete Matrices sind beispielsweise dadurch erhältlich, dass man die unbeschichtete Matrix in eine Lösung taucht, die das für die Beschichtung vorgesehene Protein oder Proteingemisch enthält, und anschließend die mit der Lösung befeuchtete Matrix trocknet. Dabei ist es in der Regel so, dass in Abhängigkeit von den Abmessungen des zu beschichtenden Matrixkörpers die Lösung vor allem die äußeren Bereiche des Matrixkörpers benetzt, während in das Innere des Matrixkörpers vergleichsweise weniger Lösung vordringt. Dies kann zur Folge haben, dass eine gleichmäßige Beschichtung der gesamten Matrixoberfläche nicht resultiert, sondern die Beschichtungsdichte von außen nach innen abnimmt.

Alternativ oder zusätzlich zu einer Beschichtung können biologisch aktive Substanzen im Polymer aufgenommen oder sogar damit verknüpft sein. Hierzu gehören beispielsweise synthetische Wirkstoffe (anorganische oder organische Moleküle), Proteine, Polysaccharide und weitere Zucker, Lipide und Nukleinsäuren, welche z.B. das Zellwachstum, die Zellmigration, die Zellteilung, die Zelldifferenzierung und/oder das Gewebewachstum beeinflussen, bzw. therapeutische, prophylaktische oder diagnostische Wirkungen besitzen. Zu nennen sind beispielsweise gefäßaktive Wirkstoffe, neuroaktive Wirkstoffe, Hormone, Wachstumsfaktoren, Cytokine, Steroide, Antikoagulanzien, entzündungshemmende Wirkstoffe, immunmodulierende Wirkstoffe, zytotoxische Wirkstoffe, Antibiotika und antivirale Wirkstoffe.

Ein Verfahren zur Herstellung einer porösen Matrix auf Basis eines biologisch verträglichen Polymers oder Polymergemisches ist **dadurch gekennzeichnet, dass** man ein Gemisch aus Polymerpartikein und Kochsalzpartikeln mit definierter Korngröße kompaktiert und anschließend das Kochsalz herauslöst.

Polymerpartikel mit einer Korngröße im Bereich von etwa 20 bis 950 µm, vorteilhafterweise im Bereich von etwa 20 bis 760 µm und insbesondere im Bereich von etwa 108 bis 250 µm und Kochsalzpartikel mit einer Korngröße im Bereich von etwa 90 bis 670 µm, vorteilhafterweise im Bereich von etwa 110 bis 520 µm und insbesondere im Bereich von etwa 250 bis 425 µm haben sich zur Einstellung der gewünschten Porengrößen bzw. Porengrößenverteilung als zweckmäßig erwiesen. Ferner hat sich ein Gewichtsverhältnis von Polymerpartikeln zu Kochsalzpartikeln im Bereich von 1:100 bis 1:10, vorteilhafterweise im Bereich von 1:50 bis 1:15 und insbesondere im Bereich von etwa 1:20 bis 1:18 zur Einstellung der gewünschten Porosität als zweckmäßig erwiesen.

Es hat sich weiterhin als zweckmäßig erwiesen, Salz und Polymer mit einer bestimmten Korngrößenverteilung zu verwenden. Was das zur Herstellung der Matrix verwendete Kochsalz angeht, so ist es günstig, wenn der Anteil an Salz mit einer Korngröße von 250 µm bis 320 µm etwa 15 % bis 50 %, vorteilhafterweise etwa 18 % bis 42 % und bevorzugterweise etwa 22 % bis 28 %; der Anteil an Salz mit einer Korngröße von 330 µm bis 380 µm etwa 20 % bis 65 %, vorteilhafterweise etwa 30 % bis 52 % und bevorzugterweise etwa 42 % bis 46 %; und der Anteil an Salz mit einer Korngröße von 390 µm bis 425 µm etwa 15 % bis 62 %, vorteilhafterweise etwa 25 % bis 42 %, und bevorzugterweise etwa 29 % bis 33 % beträgt, wobei sich die Prozentangaben auf das zur Herstellung insgesamt verwendete Gewicht an Salz beziehen. Anteile mit Korngrößen ober- und/oder unterhalb der angegebenen Bereiche sind damit nicht ausgeschlossen.

Einer speziellen Ausführungsform zufolge hat es sich als günstig erwiesen, wenn der Anteil an Kochsalzpartikeln mit einer Korngröße von 108 µm bis 140 µm 1 bis 15 Gew.-%, vorzugsweise 4 bis 12 Gew.-% und insbesondere 7 bis 9 Gew.-%, der Anteil an Salz mit einer Korngröße von 145 µm bis 180 µm 1 bis 11 Gew.%, vorzugsweise 3 bis 9 Gew.-% und insbesondere 5 bis 7 Gew.-%, der Anteil an Salz mit einer Korngröße von 185 µm bis 220 µm 3 bis 21 Gew.-%, vorzugsweise 7 bis 17 Gew.-% und insbesondere 10 bis 14 Gew.-%, der Anteil an Salz mit einer Korngröße von 225 µm bis 250 µm 1 bis 11 Gew.-%, vorzugsweise 3 bis 9 Gew.-% und insbesondere 5 bis 7 Gew.-%, der Anteil an Salz mit einer Korngröße von 250 µm bis 320 µm 15 bis 50 Gew.%, vorzugsweise 18 bis 42 Gew.-% und insbesondere 22 bis 28 Gew.-%, der Anteil an Salz mit einer Korngröße von 330 µm bis 380 µm 15 bis 50 Gew.-%, vorzugsweise 18 bis 42 Gew.-% und insbesondere 22 bis 28 Gew.-%, und der Anteil an Salz mit einer Korngröße von 390 µm bis 425 µm 5 bis 29 Gew.-%, vorzugsweise 10 bis 24 Gew.-% und insbesondere 15 bis 19 Gew.-% beträgt.

Was das zur Herstellung der Matrix verwendete Polymer angeht, so ist es günstig, wenn der Anteil an Polymer mit einer Korngröße von 108 µm bis 140 µm etwa 5 % bis 50 %, vorteilhafterweise etwa 10 % bis 30 % und bevorzugterweise etwa 14 % bis 18 %; der Anteil an Polymer mit einer Korngröße von 145 µm bis 180 µm etwa 10 % bis 55 %, vorteilhafterweise etwa 15 % bis 40 % und bevorzugterweise etwa 20 % bis 24; der Anteil an Polymer mit einer Korngröße von 185 µm bis 220 µm etwa 18 % bis 88 %, vorteilhafterweise etwa 32 % bis 76 % und bevorzugterweise etwa 43 % bis 49 %, und der Anteil an Polymer mit einer Korngröße von 225 µm bis 250 µm etwa 5 % bis 45 %, vorteilhafterweise etwa 10 % bis 28 % und bevorzugterweise etwa 14 % bis 18 % beträgt, wobei sich die Prozentangaben auf das zur Herstellung insgesamt verwendete Gewicht an Polymer beziehen.

Um Salz- bzw. Polymerpartikel der gewünschten Korngrößenverteilung zu erhalten, ist es in der Regel zweckmäßig, handelsübliche Ware zunächst zu zerkleinern. Dies kann in dafür gebräuchlichen Vorrichtungen, z.B. Schlagwerken oder Mühlen, erfolgen. Bestimmend für die gewünschte Korngrößenverteilung ist allerdings das anschließende Aussieben mit Hilfe gebräuchlicher Analysesiebe.

Das Kompaktieren erfolgt vorzugsweise durch Einwirkung von Druck. Dazu kann man das Polymer/Kochsalz-Gemisch in einer herkömmlichen Hydraulikpresse bei einem Stempeldruck im Bereich von etwa 780 psi bis 1450 psi, vorteilhafterweise im Bereich von etwa 840 psi bis 1230 psi und insbesondere im Bereich von etwa 900 psi bis 1100 psi verpressen. Es hat sich als zweckmäßig erwiesen, den Druck etwa 10 s bis 360 s, vorteilhafterweise etwa 40 s bis 180 s und insbesondere etwa 50 s bis 70 s bei Temperaturen im Bereich von 18 °C bis 25 °C einwirken zu lassen.

Das Herauslösen des Kochsalzes gelingt beispielsweise mit Wasser oder wässrigen Lösungen. So kann man das kompaktierte Gemisch (Matrixrohling) etwa 1 h bis 80 h, vorteilhafterweise etwa 12 h bis 62 h und insbesondere etwa 36 h bis 60 h wässern.

Zudem ist es von Vorteil, wenn das kompaktierte Gemisch vor dem Herauslösen des Kochsalzes zunächst in einer CO₂-Atmosphäre gelagert wird. So kann man das kompaktierte Gemisch beispielsweise bei einem CO₂-Druck im Bereich von etwa 140 psi bis 1650 psi, vorteilhafterweise im Bereich von etwa 360 psi bis 1120 psi und insbesondere im Bereich von etwa 800 psi bis 900 psi begasen, wobei sich hierbei Zeiten im Bereich von etwa 1 h bis 180 h, vorteilhafterweise im Bereich von etwa 3 h bis 60 h und insbesondere im Bereich von etwa 12 h bis 36 h als zweckmäßig erwiesen haben. Danach verringert man den Druck, wobei die Druckabsenkungsgeschwindigkeit Einfluss auf die Porenbildung hat. Wenngleich die Verwendung von CO₂ bevorzugt ist, können andere Gase, wie Luft, Stickstoff, Helium, Neon, Krypton, Argon, Xenon oder Sauerstoff ebenfalls geeignet sein.

Anschließend wird das Wasser oder die wässrige Lösung zwecks Trocknung in an sich bekannter Weise entfernt. Dazu kann man die Matrix beispielsweise auf Saugpapier legen.

Einer bevorzugten Ausführungsform zufolge setzt man dem Gemisch aus Polymerpartikeln und Kochsalzpartikeln eine Polymerlösung zu und entfernt das Lösungsmittel, bevor man kompaktiert. Dabei können Polymerpartikel und Polymerlösung auf dem gleichen Polymer basieren. Es kann sich aber auch um unterschiedliche Polymere, insbesondere mit unterschiedlicher biologischer Abbaubarkeit handeln. Der Einsatz von Polymerlösung hat den Vorteil, dass quasi Stützpfeiler in die Matrix eingezogen werden, wodurch sich die mechanischen Eigenschaften der Matrix verbessern lassen. Eine solche Matrix weist insbesondere eine geringere Neigung auf zu zerkrümeln.

Das verwendete Lösungmittel sollte das Polymer, nicht aber das Salz lösen. Dadurch ist gewährleistet, dass die porogenen Eigenschaften des Salzes nicht oder nur unwesentlich beeinflusst werden. Aceton, Ethylacetat, Methylenchlorid, Chloroform, Hexafluorisopropanol, chlorierte und fluorierte, aliphatische und aromatische Kohlenwasserstoffe, Tetrahydrofuran, Ethylmethylketon, Diethylketon sowie Gemische davon eignen sind beispielsweise zum Lösen der vorstehend genannten Polymere. Zum Lösen von Poly(glykolsäure), Poly(milchsäure) oder Poly(glykolsäuremilchsäure) und mit Blick auf die medizinische Verwendung eignet sich insbesondere Chloroform.

Gibt man die Polymerlösung und das Polymerpartikel/Salzpartikel-Gemisch zusammen, entsteht zunächst eine rührbarer Brei, der dann unter Entfernung des Lösungsmittel rasch fest wird. Konzentration des Polymers in der Lösung sind zweckmäßigerweise so zu wählen, dass einerseits das Polymer vollständig gelöst ist, andererseits das Lösungsmittel rasch entfernt werden kann ohne die Polymerpartikel in nennenswertem Umfang anzulösen.

Als günstig hat sich ein Gewichtsverhältnis von Polymerpartikeln zu gelöstem Polymer 10:1 bis 1:100, vorteilhafterweise 2:1 bis 1:25 und insbesondere 1:1 bis 1:10 erwiesen.

Was das Gewichtsverhältnis von Polymerpartikeln zu Kochsalzpartikein angeht, so kann im Rahmen dieser Ausführungsform ein zugunsten von Kochsalz höheres Gewichtsverhältnis von bis zu 1:200, 1:500 oder 1:1000 gewählt werden, wobei das Gewichtsverhältnis von Gesamtpolymer zu Kochsalz nach wie größer als 1:100 ist. Auf diese Art und Weise gelingt es, Porositäten oberhalb von 98 % einzustellen.

Bei dem vorstehend beschriebenen Verfahren dient das Kochsalz als porogenes Material, womit definitionsgemäß ein festes oder zumindest halbfestes Material gemeint ist, das mit dem matrixbildenden Polymer zunächst zu einem Gemisch vereinigt und dann aus dem Gemisch wieder entfernt wird, wodurch Hohlräume (Poren) entstehen. Dazu ist es zweckmäßig, dass das porogene Material in wenigstens einem Lösungsmittel löslich und in wenigstens einem weiteren Lösungsmittel im Wesentlichen unlöslich ist. Im Wesentlichen unlöslich ist ein Material insbesondere dann, wenn es unter den Verarbeitungsbedingungen, d.h. in der Regel bei Temperaturen im Bereich von 18 °C bis 25 °C und unter Normaldruck, zu weniger als 30 Gew.-%, vorzugsweise zu weniger als 20 Gew.-%, insbesondere zu weniger als 10 Gew.-%, beispielsweise zu weniger als 5, 4, 3, 2 und 1 Gew.-% löslich ist.

Struktur und Eigenschaften der resultierenden Matrices werden wesentlich durch das zu ihrer Herstellung verwendete porogene Material bestimmt. Dabei spielen nicht nur die Art des porogenen Materials, sondern vor allem die Korngrößenverteilung der porogenen Partikel eine Rolle. So gilt im Allgemeinen, dass mit zunehmender Korngröße nicht nur die Porengröße, sondern auch die Konnektivität, d.h. das Netzwerk miteinander kommunizierender Hohlräume, zunimmt. Dieses Netzwerk, auch Makrostruktur oder makroporöse Struktur genannt, ist zu unterscheiden von den durch Schäumen erhältlichen Poren, die in der Regel geschlossen sind und daher eine als Mikrostruktur oder mikroporös bezeichnete Struktur ausbilden.

Besondere Matrices sind demnach erhältlich nach einem Verfahren zur Herstellung einer porösen Matrix auf Basis eines biologisch verträglichen Polymers oder Polymergemisches, dass dadurch gekennzeichnet ist, dass man ein Gemisch aus Polymerpartikeln, Partikeln eines porogenen Materials und einer Polymerlösung kompaktiert und anschließend das porogene Material herauslöst.

Dieses Verfahren ist grundsätzlich nicht auf die zuvor beschriebenen Merkmale beschränkt. So kann das Polymer ausgewählt sein unter Polyanhydriden, Poly(orthoestern), Poly(α-hydroxyestern), Poly(esteramiden), Polyamiden, Poly(esterethern), Polycarbonaten, Polyalkylenen, Polyalkylenglykolen, Polyalkylenoxiden, Polyalkylenterephthalaten, Polyvinylalkoholen, Polyvinylethern, Polyvinylestern, Polyvinylhalogeniden, Polyvinylpyrrolidonen, Polysiloxanen, Polystyrolen, Polyurethanen, derivatisierten Cellulosen, (Meth)acrylsäurepotymeren und -copolymeren. Das porogene Material ist vorzugsweise ausgewählt unter wasserlöslichen Salzen, z.B. Natriumchlorid, Kaliumchlorid, Natriumfluorid, Kaliumfluorid, Natriumiodid, Kaliumiodid, Natriumnitrat, Natriumsulfat Natriumcitrat, Natriumtartrat, Zuckern (z.B. Saccharose, Fructose, Glucose) und Gemischen davon, es kann sich aber auch um wachsartige Substanzen, wie Paraffine, Bienenwachs und ähnliches handeln. Polymer, porogenes Material und das zur Bildung der Lösung verwendete Lösungsmittel sind grundsätzlich so aufeinander abzustimmen, dass die Lösung Polymer in gelöster und Polymerpartikel in fester Form enthält sowie das porogene Material im wesentlichen nicht löst.

Die mit den vorstehend beschriebenen Verfahren erhältlichen Matrices werden erfindungsgemäß bevorzugt eingesetzt.

Erfindungsgemäß zum Testen von Substanzen verwendet werden Gewebeäquivalente, die wenigstens eine der vorstehend beschriebenen Matrices und wenigstens eine Zelle umfassen. Je nach Verwendungszweck können die Zellen dabei insbesondere ausgewählt sein unter Leberzellen, Pankreaszellen, Fettzellen, Darmzellen, Hautzellen, Gefäßzellen, Nervenzellen, Muskelzellen, Schilddrüsenzellen und Zahnwurzelzellen. Besondere Ausführungsformen erfindungsgemäßer Gewebeäquivalente betreffen Leberzellen und Pankreaszellen.

Erfindungsgemäß zum Testen von Substanzen bevorzugt verwendet werden Gewebeäquivalente, die wenigstens eine Matrix auf Basis eines biologisch verträglichen Polymers und Zellen wenigstens zweier Zelltypen umfassen, wobei die Zellen des ersten Zelltyps Hepatozyten und die Zellen des zweiten Zelltyps Langerhans'sche Inselzellen sind.

Je nach Verwendungszweck, d.h. insbesondere der zu erfüllenden Funktion, sind bestimmte Verhältnisse von Hepatozyten zu Langerhans'schen Inselzellen von Vorteil. So betrifft eine Ausführungsform der Erfindung die Verwendung von Gewebeäquivalenten, die die endokrinen Eigenschaften eines äquivalenten Pankreasorgans zeigen. Hierfür hat sich ein Verhältnis von Hepatozyten zu Langerhans'schen Inselzellen von etwa 10⁶ : 3000 als vorteilhaft erwiesen. Eine weitere Ausführungsform der Erfindung betrifft Verwendung von Gewebeäquivalenten, die Stoffwechselfunktionen einer Leber vollziehen. Hierfür hat sich ein Verhältnis von Hepatozyten zu Langerhans'schen Inselzellen von etwa 10⁶ : 3-200, vorteilhafterweise von 10⁶ : 10-100, insbesondere von 10⁶: 20-80 und besonders bevorzugt von etwa 10⁶ : 35-45 als zweckmäßig erwiesen.

Es sei angemerkt, dass derartige Gewebeäquivalente in der Regel neben Hepatozyten und Langerhans'schen Inselzellen weitere Zellen beinhalten, nämlich insbesondere weitere Leber- und Pankreaszellen, die bei der Zellisolation mit anfallen.

Die zur Besiedlung erfindungsgemäßer Matrices zu verwendenden Zellen oder Zellgemische können in an sich bekannter Weise gewonnen werden. So kann man beispielsweise einem Individuum ein geeignetes Gewebe, beispielsweise ein Stück Leber oder Pankreas, entnehmen und in geeigneter Weise für die Beimpfung und *in vitro*-Kultur der Matrix vorbereiten. Hierbei ist es von Bedeutung, dass die Zellen eine möglichst hohe Vitalitätsrate aufweisen.

Gewinnt man Leberzellen aus Lebergewebe, ist zu beachten, dass die Leberzellen von einer starken Bindegewebsschicht umgeben sein können. Um die Leberzellen mit einem möglichst hohen Anteil vitaler Zellen isolieren zu können, werden erfindungsgemäß Lösungen bestimmter Zusammensetzung verwendet.

Insbesondere kann man eine NaCl, KCI und HEPES enthaltende, wässrige Zusammensetzung A mit einem pH-Wert von etwa 7,4 sowie zum Perfundieren eines Leber- oder Pankreasstücks verwenden. Insbesondere enthalten 1000 ml dieser Lösung etwa 8,3 g NaCl, 0,5 g KCI und 2,38 g HEPES. Das Perfundieren erfolgt vorzugsweise bei einer Temperatur von etwa 37 °C und einer Flussrate von etwa 30 ml/min. Wenige Minuten, insbesondere etwa 5 bis 120 Minuten, beispielsweise etwa 7 Minuten, reichen aus, um bei der vorstehend genannten Flussrate das Gewebestück ausreichend zu perfundieren.

Alternativ dazu kann man auch eine Ethylenglykoltetraessigsäure (EGTA) enthaltende, wässrige Zusammensetzung A' zum Perfundieren eines Leber- oder Pankreasstücks verwenden.

Des weiteren kann man eine wässrige Zusammensetzung B mit einem pH-Wert von etwa 7,3 bis 7,4, vorzugsweise etwa 7,35, welche NaCl, KCI, HEPES, CaCl₂, Collagenase und Trypsininhibitor enthält, sowie zum Perfundieren eines Leber- oder Pankreasstücks verwenden. Vorzugweise enthalten 1000 ml der Lösung 8,3 g NaCl, 0,5 g KCI, 2,38 g HEPES, 0,7 g CaCl₂ x 2 H₂O 500 mg Collagenase H und 7,5 mg Trypsininhibitor. Auch in diesem Fall hat sich das Perfundieren bei etwa 37 °C und einer Flussrate von etwa 30 ml/min als zweckmäßig erwiesen. Wenige Minuten, insbesondere etwa 5 bis 10 Minuten, beispielsweise etwa 6 bis 7 Minuten, reichen aus, um das Gewebestück hinreichend zu perfundieren.

Alternativ dazu kann man auch eine wässrige Zusammensetzung B' zum Perfundieren eines Leber- oder Pankreasstücks verwenden, welche Kollagenase und Hyaluronidase enthält. Vorzugsweise enthalten 1000 ml der Lösung 5 bis 10 U/ml Kollagenase und 5 bis 10 U/ml Hyaluronidase.

Es ist für die Vitalität der zu gewinnenden Zellen von Vorteil, wenn das Gewebestück zunächst mit Zusammensetzung A und anschließend mit Zusammensetzung B behandelt wird. Alternativ dazu kann zunächst eine Zusammensetzung A' und dann eine Zusammensetzung B' verwendet werden.

Im Anschluss an die Perfusion kann das Gewebestück dann frei präpariert werden und in einem geeigneten Medium, beispielsweise Williams-Medium E, vorsichtig aufgeschüttelt werden. Enthält die resultierende Zellsuspension noch gröbere Zelltrümmer, können diese in an sich bekannter Weise entfernt werden, beispielsweise indem man die Zellsuspension über ein Nylonnetz (200 µm) filtriert. Die Zellen des Filtrats können dann vorsichtig pelletiert werden, wobei sich eine dreiminütige Zentrifugation bei 50 g und 4 °C als vorteilhaft erwiesen hat.

Die Auftragung der gewonnenen Zellen auf die Matrices erfolgt in an sich bekannter Weise. In der Regel werden die Zellen als zellhaltige Lösung auf die Matrix aufgetragen und anschließend - üblicherweise unter Zellkulturbedingungen - inkubiert, bis Zellen an der Matrix anhaften. Werden mehr als ein Zelltyp, beispielsweise Hepatocyten und Langerhans'sche Inselzellen, auf eine Matrix aufgetragen, können die verschiedenen Zelltypen im Prinzip gemeinsam oder aber nacheinander aufgetragen werden. Einer besonderen Ausführungsform zufolge trägt man zunächst Langerhans'sche Inselzellen und anschließend Hepatocyten auf, wobei man nach dem Auftragen jeweils inkubiert, bis zumindest ein Teil der Zellen an der Matrix anhaftet.

Erfindungsgemäße Matrices und Gewebeäquivalente weisen entscheidende Vorteile auf. So ermöglichen die inneren Dimensionen der Matrices eine effiziente Besiedlung mit Zellen. Die Matrices sind einerseits frei verformbar und bieten andererseits ausreichend Stabilität und Rigidität. Die erfindungsgemäßen Matrices können hergestellt werden, ohne physiologisch bedenkliche Lösungsmittel, beispielsweise Formaldehyd, verwenden zu müssen, so dass kein spezielles Verfahren zur Elimination der Lösungsmittel erforderlich ist und die Gefahr verbleibender Restmengen dieser Lösungsmittel nicht besteht.

Erfindungsgemäße Gewebeäquivalente weisen vielfältige Verwendungsmöglichkeiten auf. Hiervon sind insbesondere Verwendungen in vitro zu nennen. Ein weiterer Gegenstand der vorliegenden Erfindung sind daher die erfindungsgemäßen Gewebeäquivalente zur Verwendung in vitro.

Eine besondere Verwendung in diesem Bereich basiert auf dem Aufbau von Gewebe (Tissue Engineering). Dabei dienen die erfindungsgemäßen Matrices quasi als Gerüst (Scaffold), in das Zellen einwandern und/oder sich anheften.

Dazu kann man die Matrices beispielsweise in vitro mit den gewünschten Zellen beimpfen, z.B. mit einer zellhaltigen Lösung versetzen und inkubieren, bis Zellen sich an die Matrix angeheftet haben. Eine solche Matrix mit daran anhaftenden Zellen (hier mit Gewebeäquivalente bezeichnet) kann dann weiteren Verfahrensmaßnahmen, beispielsweise weiterer Kultivierung, gegebenenfalls unter Einwirkung von Wirkstoffen, z.B. zur weiteren Expansion der Zellen oder zur Modulierung ihrer Eigenschaften, unterzogen werden, und/oder bis zur Verwendung in geeigneter Weise, beispielsweise auf Eis oder in einem Bioflussreaktor unter Standardbedingungen, aufbewahrt werden. Im Rahmen dieser Verwendung ist es von Vorteil, die zum Testen der Substanzen bestimmten Zellen zunächst in vitro isolieren und gegebenenfalls auch expandieren zu können. Insbesondere wird dadurch das Aufbringen verschiedener Zelltypen, wie die oben beschriebenen Hepatozyten zusammen mit Langerhans'schen Inselzellen, auf eine Matrix ermöglicht.

Die folgenden Beispiele sollen die Erfindung veranschaulichen, ohne sie in ihrem Umfang einzuschränken.

### Beispiel 1

### Herstellung der Matrix

### a) Ohne Polymerlösung

Polymer-Pellets (Resomer® RG 858, erhältlich von der Firma Boehringer, Ingelheim) werden in Flüssigstickstoff gefroren und in gefrorenem Zustand geschreddert (Schlagwerk der Firma Däschle; 12000 U/min 2 min). Die geschredderten Polymerpartikel werden gesiebt. Partikel mit einer Größe von 108 µm bis 250 µm werden für die Matrixherstellung eingesetzt. Dabei besitzen 16 Gew.-% des eingesetzten Polymers eine Partikelgröße zwischen 108 µm und 140 µm, 22 Gew.-% des eingesetzten Polymers eine Partikelgröße zwischen 145 µm und 180 µm, 46 Gew.-% des eingesetzten Polymers eine Partikelgröße zwischen 185 µm und 220 µm, und 16 Gew.-% des eingesetzten Polymers eine Partikelgröße zwischen 225 µm und 250 µm. Kochsalz wird gesiebt und Kochsalzpartikel mit einer Korngröße von 250 µm bis 425 µm werden für die Matrixherstellung verwendet. Dabei besitzen 25 Gew.-% des eingesetzten Salzes eine Partikelgröße zwischen 250 µm und 320 µm, 44 Gew.-% des eingesetzten Salzes eine Partikelgröße zwischen 330 µm und 380 µm, und 31 Gew.-% des eingesetzten Salzes eine Partikelgröße zwischen 390 µm und 425 µm. 760 mg Kochsalzpartikel und 40 mg Polymerpartikel werden miteinander vermischt. Das Gemisch wird in eine Stanzform eingebracht und mit einer Hydraulikpresse bei einem Stempeldruck von 1000 psi 1 Minute gepresst. Anschließend werden die Matrixrohlinge auf einen Teflonteller gelegt und 24 Stunden in einer CO₂-Atmosphäre (850 psi) begast. Dann werden die Rohlinge 24 Stunden gewässert, um die eingeschlossenen Salzkörner aufzulösen. Schließlich werden die Matrices 12 Stunden auf Saugpapier getrocknet.

Die resultierende Polymermatrix weist eine Porosität von 95 +/- 2% und eine mittels Rasterelektronenmikroskopie bestimmte, definierte Porengröße von 250 µm +/- 120 µm auf.

### b) Mit Polymerlösung

Kochsalz (analytisch rein) wird gemahlen (Schlagwerk der Firma Däschle; 12000 U/min 2 min) und anschließend gesiebt, und Kochsalzpartikel mit einer Korngröße von 108 bis 425 µm werden für die Matrixherstellung verwendet. Dabei besitzen 8 % des eingesetzten Salzes eine Partikelgröße zwischen 108 µm und 140 µm, 6 Gew.-% des eingesetzten Salzes eine Partikelgröße zwischen 145 µm und 180 µm, 12 Gew.-% des eingesetzten Salzes eine Partikelgröße zwischen 185 µm und 220 µm, 6 Gew.-% des eingesetzten Salzes eine Partikelgröße zwischen 225 µm und 250 µm, 25 Gew.-% des eingesetzten Salzes eine Partikelgröße zwischen 250 µm und 320 µm, 26 Gew.-% des eingesetzten Salzes eine Partikelgröße zwischen 330 µm und 380 µm, und 17 Gew.-% des eingesetzten Salzes eine Partikelgröße zwischen 390 µm und 425 µm. 96 g Kochsalzpartikel werden mit 1 g der in Beispiel 1 a) beschriebenen Polymerpartikel vermischt und anschließend mit 100 ml einer Chloroformlösung, die 4 g des Polymers gelöst enthält, versetzt. Das so erhaltene Gemisch wird bei 45 °C bis 65 °C erwärmt, wodurch das Chloroform innerhalb von etwa 25 Minuten verdampft. Das verbleibende Salz-Polymer-Gemisch wird dann mit einer Hydraulikpresse bei einem Stempeldruck von 1000 psi eine Minute gepresst und anschließend 24 Stunden gewässert, um die eingeschlossenen Salzkörner aufzulösen. Anschließend wird die Matrix, wie oben beschrieben, begast und schließlich 12 Stunden auf Saugpapier getrocknet.

Die resultierende Polymermatrix weist eine Porosität von 96 % auf.

Vermischt man 98,5 g Salzpartikel mit 0,5 g Polymerpartikel und versetzt das Gemisch mit 100 ml einer Chloroform-Lösung, die 1 g Polymer enthält, erhält man eine Matrix mit einer Porosität von 99 %.

Vermischt man 99,2 g Salzpartikel mit 0,1 g Polymerpartikel und versetzt dieses Gemisch mit 100 ml einer Chloroform-Lösung, die etwa 0,9 g Polymer enthält, erhält man eine Polymermatrix mit einer Porosität von 99 %.

### Beispiel 2

### a) Beschichtung der Matrix mit Fibronectin

Die Matrix aus Beispiel 1 wird in eine 3 µg/ml Fibronectin aus Humanplasma (Sigma) enthaltende Carbonatpufferlösung mit einem pH-Wert von 9,4 getaucht. Nach etwa 60 s wird die Matrix aus der Lösung entnommen, lyophilisiert und γ-sterilisiert.

### Beispiel 3

### Zellisolation

Ein Leberstück eines humanen Spenders wird zunächst 7 Minuten bei einer Flussrate von 30 ml/min und 37 °C mit einer Lösung (8,3 g NaCl; 0,5 g KCI; 2,38 g HEPES; ad 1000 ml destilliertes Wasser; pH-Wert 7,4) perfundiert. Anschließend wird das Leberstück weitere 6 bis 7 min bei einer Flussrate von 30 ml/min und 37 °C mit einer Collagenase-Trypsin-Inhibitor-Lösung (8,3 g NaCl; 0,5 g KCl; 2,38 g HEPES; 0,7 g CaCl₂ x 2 H₂O; 500 mg Collagenase (Collagenase H, Boehringer Mannheim, Mannheim, Deutschland); 7,5 mg Trypsin-Inhibitor (ICN, Eschwege, Deutschland); ad 1000 ml destilliertes Wasser; pH-Wert 7,35) perfundiert. Nach Beendigung der Perfusion wurde das Leberstück frei präpariert und in Williams-Medium E vorsichtig aufgeschüttelt. Die Zellsuspension wird filtriert (Nylonnetz; 200 µm) und anschließend mit Williams-Medium E gewaschen. Anschließend werden die Zellen bei 3 min bei 50 g und 4 °C zentrifugiert. Die mit Trypan-Blau bestimmte Vitalität der Zellen beträgt 95 %.

In gleicher Weise werden Langerhans'sche Inselzellen aus einem Pankreasstück isoliert.

### Beispiel 4

### Zellbesiedlung

Die in Beispiel 2 beschichteten Matrices werden im ersten Schritt mit Langerhans'schen Inselzellen inkubiert, welche gemäß Beispiel 3 isoliert wurden.

Dazu wurden 3000 Inselzellen pro ml in einem Lösungsgemisch aus M199 und FKS (Volumenverhältnis von 19:1) suspendiert. Die Zellzahl wird bestimmt, indem man sie unter einem inversen Olympus-Mikroskop in einem 0,25-mm-Zählrohr ausgezählt. Dann werden 8 ml bis 10 ml dieser Lösung mit einer Pipette auf die Matrix aufgebracht. Die überschüssige Lösung, welche nicht in der Matrix verbleibt, wird verworfen. Die so behandelte Matrix wird anschließend zur Anheftung der Zellen 4 Stunden in den Zellkulturbrutschrank gestellt. Anschließend wird eine Lösung aus Williams Medium E, die pro ml eine nicht gereinigte Leberzellsuspension mit etwa 5,0 x 10⁷ vitalen Hepatocyten und etwa 1,0 x 10⁸ nichtperenchymatösen Leberzellen enthält, auf die Matrix aufgebracht. Es werden 8 ml bis 12 ml Lösung mit einer Pipette aufgetragen; die nicht von der Matrix aufgenommene überschüssige Lösung wird verworfen.

### Beispiel 5

### Induktion eines Cytochroms P450 durch Benzol

Ein gemäß Beispiel 4 erhaltenes Gewebeäquivalent (etwa 124 mm x 45 mm x 5 mm) wird in 8 Streifen zerlegt. Die Streifen gibt man in etwa 1 I Williams-Medium E, das bewegt wird. Ein ausreichender Gasaustausch wird gewährleistet und die Temperatur des Mediums bei 37 °C gehalten.

Nachdem sich das System äquilibriert hat, entnimmt man einen ersten Streifen Gewebeäquivalent und bestimmt hieran über das EFCOD-Testverfahren die CYP2E1-Aktivität. Mit besagtem Verfahren wird die CYP2E1-vermittelte O-Desalkylierung von 7-Ethoxy-4-trifluormethylcoumarin zu 7-Hydroxy-4-trifluormethylcoumarin bestimmt.

Anschließend setzt man dem Medium so viel Benzol zu, das die Benzolkonzentration bei 0,005 mM liegt. Man lässt das Benzol mehrere Stunden einwirken, wobei das Medium nach wie vor bewegt und der Gasaustausch gewährleistet wird. Dann bestimmt man in der oben beschriebenen Weise erneut die CYP2E1-Aktivität.

Anschließend erhöht man die Benzolkonzentration auf 0,01, 0,02, 0,05 und 0,1 mM, wobei man die CYP2E1-Aktivität nach Einwirken der jeweiligen Benzolkonzentration bestimmt.

Ein Vergleich der auf diese Weise gemessenen CYP2E1-Aktivitäten zeigt einen deutlichen Anstieg der Grundaktivität mit zunehmender Benzolkonzentration - eine Veränderung, welche die an sich bekannte Lebertoxizität von Benzol wiederspiegelt.

## Patentansprüche

1. Verfahren zum Testen einer oder mehrerer Substanzen, wobei man ein Gewebeäquivalent kultiviert; die Substanz(en) auf das Gewebeäquivalent einwirken lässt; und bestimmt, ob das Einwirken der Substanz(en) zu einer Veränderung des Gewebeäquivalents und/oder der Substanz(en) geführt hat, **dadurch gekennzeichnet, dass** das Gewebeäquivalent mindestens eine Gewebszelle und eine poröse Matrix auf Basis eines biologisch verträglichen Polymers oder Polymergemisches umfasst, wobei die Matrix etwa 0,5 % bis 6 % Poren mit einem mittleren Durchmesser im Bereich von 70 bis 100 µm, etwa 2 % bis 8 % Poren mit einem mittleren Durchmesser im Bereich von 101 bis 115 µm, etwa 2 % bis 8 % Poren mit einem mittleren Durchmesser im Bereich von 116 bis 130 µm, etwa 1 % bis 7 % Poren mit einem mittleren Durchmesser im Bereich von 131 bis 300 µm, etwa 11 % bis 23 % Poren mit einem mittleren Durchmesser im Bereich von 301 bis 330 µm, etwa 4 % bis 10 % Poren mit einem mittleren Durchmesser im Bereich von 331 bis 360 µm, etwa 5 % bis 17 % Poren mit einem mittleren Durchmesser im Bereich von 361 bis 390 µm, etwa 7 % bis 19 % Poren mit einem mittleren Durchmesser im Bereich von 391 bis 420 µm, etwa 3 % bis 9 % Poren mit einem mittleren Durchmesser im Bereich von 421 bis 450 µm, etwa 12 % bis 24 % Poren mit einem mittleren Durchmesser im Bereich von 451 bis 480 µm und etwa 5 % bis 17 % Poren mit einem mittleren Durchmesser im Bereich von 481 bis 510 µm aufweist, der Porositätsgrad 93 bis 98 % beträgt und die Matrix durch Kompaktieren eines Gemisches aus Polymerpartikeln und Kochsalzpartikeln und anschließendes Herauslösen des Kochsalzes erhältlich ist.

2. Verfahren nach Anspruch 1, **dadurch gekennzeichnet, dass** die Matrix etwa 1 % bis 5 % Poren mit einem mittleren Durchmesser im Bereich von 70 bis 100 µm, etwa 3 % bis 7 % Poren mit einem mittleren Durchmesser im Bereich von 101 bis 115 µm, etwa 3 % bis 7 % Poren mit einem mittleren Durchmesser im Bereich von 116 bis 130 µm, etwa 2 % bis 6 % Poren mit einem mittleren Durchmesser im Bereich von 131 bis 300 µm, etwa 13 % bis 21 % Poren mit einem mittleren Durchmesser im Bereich von 301 bis 330 µm, etwa 5 % bis 9 % Poren mit einem mittleren Durchmesser im Bereich von 331 bis 360 µm, etwa 7 % bis 15 % Poren mit einem mittleren Durchmesser im Bereich von 361 bis 390 µm, 9 % bis 17 % Poren mit einem mittleren Durchmesser im Bereich von 391 bis 420 µm, etwa 4 % bis 8 % Poren mit einem mittleren Durchmesser im Bereich von 421 bis 450 µm, etwa 14 % bis 22 % Poren mit einem mittleren Durchmesser im Bereich von 451 bis 480 µm und etwa 7 % bis 15 % Poren mit einem mittleren Durchmesser im Bereich von 481 bis 510 µm aufweist.

3. Verfahren nach Anspruch 1, **dadurch gekennzeichnet, dass** die Matrix etwa 2 % bis 4 % Poren mit einem mittleren Durchmesser im Bereich von 70 bis 100 µm, etwa 4 % bis 6 % Poren mit einem mittleren Durchmesser im Bereich von 101 bis 115 µm, etwa 4 % bis 6 % Poren mit einem mittleren Durchmesser im Bereich von 116 bis 130 µm, etwa 3 % bis 5 % Poren mit einem mittleren Durchmesser im Bereich von 131 bis 300 µm, etwa 15 % bis 19 % Poren mit einem mittleren Durchmesser im Bereich von 301 bis 330 µm, etwa 6 % bis 8 % Poren mit einem mittleren Durchmesser im Bereich von 331 bis 360 µm, etwa 9 % bis 13 % Poren mit einem mittleren Durchmesser im Bereich von 361 bis 390 µm, etwa 11 % bis 15 % Poren mit einem mittleren Durchmesser im Bereich von 391 bis 420 µm, etwa 5 % bis 7 % Poren mit einem mittleren Durchmesser im Bereich von 421 bis 450 µm, etwa 16 % bis 20 % Poren mit einem mittleren Durchmesser im Bereich von 451 bis 480 µm und etwa 9 % bis 13 % Poren mit einem mittleren Durchmesser im Bereich von 481 bis 510 µm aufweist.

4. Verfahren nach Anspruch 1, **dadurch gekennzeichnet, dass** die Matrix etwa 3 % Poren mit einem mittleren Durchmesser im Bereich von 70 bis 100 µm, etwa 5 % Poren mit einem mittleren Durchmesser im Bereich von 101 bis 115 µm, etwa 5 % Poren mit einem mittleren Durchmesser im Bereich von 116 bis 130 µm, etwa 4 % Poren mit einem mittleren Durchmesser im Bereich von 131 bis 300 µm, etwa 17 % Poren mit einem mittleren Durchmesser im Bereich von 301 bis 330 µm, etwa 7 % Poren mit einem mittleren Durchmesser im Bereich von 331 bis 360 µm, etwa 11 % Poren mit einem mittleren Durchmesser im Bereich von 361 bis 390 µm, etwa 13 % Poren mit einem mittleren Durchmesser im Bereich von 391 bis 420 µm, etwa 6 % Poren mit einem mittleren Durchmesser im Bereich von 421 bis 450 µm, etwa 18 % Poren mit einem mittleren Durchmesser im Bereich von 451 bis 480 µm und etwa 11 % Poren mit einem mittleren Durchmesser im Bereich von 481 bis 510 µm aufweist.

5. Verfahren nach einem der Ansprüche 1 bis 4, **dadurch gekennzeichnet, dass** das biologisch verträgliche Polymer ein biologisch abbaubares Polymer ist, das ausgewählt ist unter natürlichen Polymeren, wie Albumin, Fibrinogen, Collagen, Gelatine, Chitin, Chitosan, Agarose, Alginat und synthetischen Polymeren, wie Polyanhydriden, Poly(ε-caprolacton) und Poly(α-hydroxyestern).

6. Verfahren nach Anspruch 5, **dadurch gekennzeichnet, dass** das biologisch abbaubare Polymer Poly(glycotsäure-milchsäure) mit einem Milchsäure-Anteil von etwa 85 mol-% und einem Glycolsäure-Anteil von etwa 15 mol-% ist.

7. Verfahren nach einem der Ansprüche 1 bis 6, **dadurch gekennzeichnet, dass** die Oberfläche der Matrix mit wenigstens einem extrazellulären Matrixprotein beschichtet ist, das ausgewählt ist unter Collagenen, Laminin und Fibronectin.

8. Verfahren nach Anspruch 7, **dadurch gekennzeichnet, dass** die Beschichtung ein Gemisch aus Collagen vom Typ I, Laminin und Collagen vom Typ IV enthält.

9. Verfahren nach einem der vorhergehenden Ansprüche, **dadurch gekennzeichnet, dass** die poröse Matrix auf Basis eines biologisch verträglichen Polymers oder Polymergemisches erhältlich ist, indem man ein Gemisch aus Polymerpartikeln mit einer Korngröße im Bereich von etwa 20 bis 950 µm, vorteilhafterweise im Bereich von etwa 50 bis 760 µm und insbesondere im Bereich von etwa 108 bis 250 µm, und Kochsalzpartikeln mit einer Korngröße im Bereich von etwa 90 bis 670 µm, vorteilhafterweise im Bereich von etwa 110 bis 520 µm und insbesondere im Bereich von etwa 250 bis 425 µm, kompaktiert und anschließend das Kochsalz herauslöst.

10. Verfahren nach Anspruch 9, **dadurch gekennzeichnet, dass** das Kochsalzpartikelgemisch aus 15 bis 50 Gew.%, vorzugsweise 18 bis 42 Gew.-% und insbesondere 22 bis 28 Gew.-% Partikel mit einer Korngröße von 250 µm bis 320 µm, 20 bis 65 Gew.%, vorzugsweise 30 bis 52 Gew.% und insbesondere 42 bis 46 Gew.-% Partikel mit einer Korngröße von 330 bis 380 µm, und 15 bis 62 Gew.%, vorzugsweise 25 bis 42 Gew.% und insbesondere 29 bis 33 Gew.% Partikel mit einer Korngröße von 390 µm bis 425 µm besteht.

11. Verfahren nach Anspruch 9, **dadurch gekennzeichnet, dass** das Kochsalzpartikelgemisch aus 1 bis 15 Gew.%, vorzugsweise 4 bis 12 Gew.% und insbesondere 7 bis 9 Gew.-% Partikel mit einer Korngröße von 108 µm bis 140 µm, 1 bis 11 Gew.%, vorzugsweise 3 bis 9 Gew.% und insbesondere 5 bis 7 Gew.-% Partikel mit einer Korngröße von 145 µm bis 180 µm, 3 bis 21 Gew.-%, vorzugsweise 7 bis 17 Gew.-% und insbesondere 10 bis 14 Gew.% Partikel mit einer Korngröße von 185 µm bis 220 µm, 1 bis 11 Gew.%, vorzugsweise 3 bis 9 Gew.% und insbesondere 5 bis 7 Gew.% Partikel mit einer Korngröße von 225 µm bis 250 µm, 15 bis 50 Gew.%, vorzugsweise 18 bis 42 Gew.-% und insbesondere 22 bis 28 Gew.-% Partikel mit einer Korngröße von 250 µm bis 320 µm, 15 bis 50 Gew.%, vorzugsweise 18 bis 42 Gew.-% und insbesondere 22 bis 28 Gew.-% Partikel mit einer Korngröße von 330 µm bis 380 µm, und 5 bis 29 Gew.%, vorzugsweise 10 bis 24 Gew.-% und insbesondere 15 bis 19 Gew.-% Partikel mit einer Korngröße von 390 µm bis 425 µm besteht.

12. Verfahren nach einem der Ansprüche 9 bis 11, **dadurch gekennzeichnet, dass** das Polymerpartikelgemisch aus 5 bis 50 Gew.-%, vorzugsweise 10 bis 30 Gew.-% und insbesondere 14 bis 18 Gew.% Partikel mit einer Korngröße von 108 µm bis 140 µm, 10 bis 55 Gew.%, vorzugsweise 15 bis 40 Gew.% und insbesondere 20 bis 24 Gew.-% Partikel mit einer Korngröße von 145 µm bis 180 µm, 18 bis 88 Gew.%, vorzugsweise 32 bis 76 Gew.-% und insbesondere 43 bis 49 Gew.% Partikel mit einer Korngröße von 185 µm bis 220 µm, und 5 bis 45 Gew.-%, vorzugsweise 10 bis 28 Gew.% und insbesondere 14 bis 18 Gew.% Partikel mit einer Korngröße von 225 µm bis 250 µm besteht.

13. Verfahren nach einem der Ansprüche 9 bis 12, **dadurch gekennzeichnet, dass** das Gewichtsverhältnis von Polymerpartikeln zu Kochsalzpartikeln 1:100 bis 1:10, vorteilhafterweise 1:50 bis 1:15 und insbesondere 1:20 bis 1:18 beträgt.

14. Verfahren nach einem der Ansprüche 9 bis 13, **dadurch gekennzeichnet, dass** man vor dem Kompaktieren dem Gemisch aus Polymerpartikeln und Kochsalzpartikeln eine Polymerlösung zusetzt und das Lösungsmittel entfernt.

15. Verfahren nach Anspruch 14, **dadurch gekennzeichnet, dass** das Lösungsmittel das Polymer, nicht aber das Salz löst.

16. Verfahren nach Anspruch 15, **dadurch gekennzeichnet, dass** das Lösungsmittel ausgewählt ist unter Aceton, Ethylacetat, Methylenchlorid, Chloroform, Hexafluorisopropanol, chlorierten und fluorierten, aliphatischen und aromatischen Kohlenwasserstoffen, Tetrahydrofuran, Ethylmethylketon, Diethylketon sowie Gemischen davon.

17. Verfahren nach Anspruch 15, **dadurch gekennzeichnet, dass** das Polymer Poly(glykolsäure), Poly(milchsäure) oder Poly(glykolsäuremilchsäure) und das Lösungsmittel Chloroform ist.

18. Verfahren nach einem der Ansprüche 14 bis 17, **dadurch gekennzeichnet, dass** das Gewichtsverhältnis von Polymerpartikeln zu gelöstem Polymer 10:1 bis 1:100, vorteilhafterweise 2:1 bis 1:25 und insbesondere 1:1 bis 1:10 beträgt.

19. Verfahren nach Anspruch 9, **dadurch gekennzeichnet, dass** das Kompaktieren durch Einwirken von Druck erfolgt.

20. Verfahren nach Anspruch 9, **dadurch gekennzeichnet, dass** man zum Herauslösen des Kochsalzes Wasser auf das kompaktierte Gemisch einwirken lässt.

21. Verfahren nach Anspruch 20, **dadurch gekennzeichnet, dass** man das Wasser wieder entfernt.

22. Verfahren nach Anspruch 9, **dadurch gekennzeichnet, dass** das kompaktierte Gemisch zunächst in einer CO₂-Atmosphäre gelagert und anschließend das Kochsalz herauslöst wird.

23. Verfahren nach einem der vorhergehenden Ansprüche, **dadurch gekennzeichnet, dass** das Gewebeäquivalent Gewebezellen wenigstens zweier Zelltypen umfasst, wobei die Zellen des ersten Zelltyps Hepatozyten und die Zellen des zweiten Zelltyps Langerhans'sche Inselzellen sind.

24. Verfahren nach Anspruch 23, **dadurch gekennzeichnet, dass** das Verhältnis von Hepatozyten zu Langerhans'schen Inselzellen etwa 10⁶ : 3000 beträgt.

25. Verfahren nach Anspruch 23, **dadurch gekennzeichnet, dass** das Verhältnis von Hepatozyten zu Langerhans'schen Inselzellen etwa 10⁶ : 3-200, vorteilhafterweise etwa 10⁶ : 10-100, insbesondere etwa10⁶ : 20-80 und besonders bevorzugt etwa 10⁶ : 35-45 beträgt.

## Claims

1. A method for testing one or more substances, wherein there is cultivated a tissue equivalent, the substance(s) is or are allowed to act on the tissue equivalent, and it is determined whether the action of the substance(s) has led to a change of the tissue equivalent and/or of the substance(s), **characterized in that** the tissue equivalent comprises at least one tissue cell and a porous matrix based on a biocompatible polymer or polymer mixture, wherein the matrix comprises approximately 0.5% to 6% of pores with a mean diameter in the range of 70 to 100 µm, approximately 2% to 8% of pores with a mean diameter in the range of 101 to 115 µm; approximately 2% to 8% of pores with a mean diameter in the range of 116 to 130 µm; approximately 1% to 7% of pores with a mean diameter in the range of 131 to 300 µm; approximately 11 % to 23% of pores with a mean diameter in the range of 301 to 330 µm; approximately 4% to 10% of pores with a mean diameter in the range of 331 to 360 µm; approximately 5% to 17% of pores with a mean diameter in the range of 361 to 390 µm; approximately 7% to 19% of pores with a mean diameter in the range of 391 to 420 µm; approximately 3% to 9% of pores with a mean diameter in the range of 421 to 450 µm; approximately 12% to 24% of pores with a mean diameter in the range of 451 to 480 µm; and approximately 5% to 17% of pores with a mean diameter in the range of 481 to 510 µm, the degree of porosity is 93 to 98% and the matrix is obtainable by compacting a mixture of polymer particles and salt and then dissolving out the salt.

2. A method according to claim 1, **characterized in that** the matrix comprises approximately 1% to 5% of pores with a mean diameter in the range of 70 to 100 µm, approximately 3% to 7% of pores with a mean diameter in the range of 101 to 115 µm; approximately 3% to 7% of pores with a mean diameter in the range of 116 to 130 µm; approximately 2% to 6% of pores with a mean diameter in the range of 131 to 300 µm; approximately 13% to 21 % of pores with a mean diameter in the range of 301 to 330 µm; approximately 5% to 9% of pores with a mean diameter in the range of 331 to 360 µm; approximately 7% to 15% of pores with a mean diameter in the range of 361 to 390 µm; approximately 9% to 17% of pores with a mean diameter in the range of 391 to 420 µm; approximately 4% to 8% of pores with a mean diameter in the range of 421 to 450 µm; approximately 14% to 22% of pores with a mean diameter in the range of 451 to 480 µm; and approximately 7% to 15% of pores with a mean diameter in the range of 481 to 510 µm.

3. A method according to claim 1, **characterized in that** the matrix comprises approximately 2% to 4% of pores with a mean diameter in the range of 70 to 100 µm, approximately 4% to 6% of pores with a mean diameter in the range of 101 to 115 µm; approximately 4% to 6% of pores with a mean diameter in the range of 116 to 130 µm; approximately 3% to 5% of pores with a mean diameter in the range of 131 to 300 µm; approximately 15% to 19% of pores with a mean diameter in the range of 301 to 330 µm; approximately 6% to 8% of pores with a mean diameter in the range of 331 to 360 µm; approximately 9% to 13% of pores with a mean diameter in the range of 361 to 390 µm; approximately 11 % to 15% of pores with a mean diameter in the range of 391 to 420 µm; approximately 5% to 7% of pores with a mean diameter in the range of 421 to 450 µm; approximately 16% to 20% of pores with a mean diameter in the range of 451 to 480 µm; and approximately 9% to 13% of pores with a mean diameter in the range of 481 to 510 µm.

4. A method according to claim 1, **characterized in that** the matrix comprises approximately 3% of pores with a mean diameter in the range of 70 to 100 µm, approximately 5% of pores with a mean diameter in the range of 101 to 115 µm; approximately 5% of pores with a mean diameter in the range of 116 to 130 µm; approximately 4% of pores with a mean diameter in the range of 131 to 300 µm; approximately 17% of pores with a mean diameter in the range of 301 to 330 µm; approximately 7% of pores with a mean diameter in the range of 331 to 360 µm; approximately 11 % of pores with a mean diameter in the range of 361 to 390 µm; approximately 13% of pores with a mean diameter in the range of 391 to 420 µm; approximately 6% of pores with a mean diameter in the range of 421 to 450 µm; approximately 18% of pores with a mean diameter in the range of 451 to 480 µm; and approximately 11 % of pores with a mean diameter in the range of 481 to 510 µm.

5. A method according to any one of claims 1 to 4, **characterized in that** the biocompatible polymer is a biodegradable polymer which is chosen from among natural polymers, such as albumin, fibrinogen, collagen, gelatin, chitin, chitosan, agarose, alginate and synthetic polymers such as polyanhydrides, poly(ε-caprolactone) and poly(α-hydroxy esters).

6. A method according to claim 5, **characterized in that** the biodegradable polymer is poly(glycolic acid/lactic acid) with a lactic acid content of approximately 85 mol% and a glycolic acid content of approximately 15 mol%.

7. A method according to one of claims 1 to 6, **characterized in that** the surface of the matrix is coated with at least one extracellular matrix protein which is chosen from among collagens, laminin and fibronectin.

8. A method according to claim 7, **characterized in that** the coating contains a mixture of type I collagen, laminin and type IV collagen.

9. A method according to one of the preceding claims, **characterized in that** the porous matrix based on a biocompatible polymer or polymer mixture can be obtained by compacting a mixture of polymer particles with a particle size in the range of approximately 20 to 950 µm, advantageously in the range of approximately 50 to 760 µm and especially in the range of approximately 108 to 250 µm, and salt particles with a particle size in the range of approximately 90 to 670 µm, advantageously in the range of approximately 110 to 520 µm and especially in the range of approximately 250 to 425 µm, and then dissolving out the salt.

10. A method according to claim 9, **characterized in that** the mixture of salt particles is composed of 15% to 50 wt%, preferably 18% to 42 wt% and especially 22% to 28 wt% of particles with a particle size of 250 µm to 320 µm, 20% to 65 wt%, preferably 30% to 52 wt% and especially 42% to 46 wt% of particles with a particle size of 330 µm to 380 µm, and 15% to 62 wt%, preferably 25% to 42 wt% and especially 29% to 33 wt% of particles with a particle size of 390 µm to 425 µm.

11. A method according to claim 9, **characterized in that** the mixture of salt particles is composed of 1 to 15 wt%, preferably 4 to 12 wt% and especially 7 to 9 wt% of particles with a particle size of 108 µm to 140 µm, 1 to 11 wt%, preferably 3 to 9 wt% and especially 5 to 7 wt% of particles with a particle size of 145 µm to 180 µm, 3 to 21 wt%, preferably 7 to 17 wt% and especially 10 to 14 wt% of particles with a particle size of 185 µm to 220 µm, 1 to 11 wt%, preferably 3 to 9 wt% and especially 5 to 7 wt% of particles with a particle size of 225 µm to 250 µm, 15 to 50 wt%, preferably 18 to 42 wt% and especially 22 to 28 wt% of particles with a particle size of 250 µm to 320 µm, 15 to 50 wt%, preferably 18 to 42 wt% and especially 22 to 28 wt% of particles with a particle size of 330 µm to 380 µm, and 5 to 29 wt%, preferably 10 to 24 wt% and especially 15 to 19 wt% of particles with a particle size of 390 µm to 425 µm.

12. A method according to one of claims 9 to 11, **characterized in that** the mixture of polymer particles is composed of 5 to 50 wt%, preferably 10 to 30 wt% and especially 14 to 18 wt% of particles with a particle size of 108 µm to 140 µm, 10 to 55 wt%, preferably 15 to 40 wt% and especially 20 to 24 wt% of particles with a particle size of 145 µm to 180 µm, 18 to 88 wt%, preferably 32 to 76 wt% and especially 43 to 49 wt% of particles with a particle size of 185 µm to 220 µm, and 5 to 45 wt%, preferably 10 to 28 wt% and especially 14 to 18 wt% of particles with a particle size of 225 µm to 250 µm.

13. A method according to one of claims 9 to 12, **characterized in that** the weight ratio of polymer particles to salt particles is 1:100 to 1:10, advantageously 1:50 to 1:15 and especially 1:20 to 1:18.

14. A method according to one of claims 9 to 13, **characterized in that** a polymer solution is added to the mixture of polymer particles and salt particles and the solvent is evaporated before compaction.

15. A method according to claim 14, **characterized in that** the solvent dissolves the polymer but not the salt.

16. A method according to claim 15, **characterized in that** the solvent is chosen from among acetone, ethyl acetate, methylene chloride, chloroform, hexafluoroisopropanol, chlorinated and fluorinated aliphatic and aromatic hydrocarbons, tetrahydrofuran, methyl ethyl ketone, diethyl ketone and mixtures thereof.

17. A method according to claim 15, **characterized in that** the polymer is poly(glycolic acid), poly(lactic acid) or poly(glycolic acid/lactic acid), and the solvent is chloroform.

18. A method according to one of claims 14 to 17, **characterized in that** the weight ratio of polymer particles to dissolved polymer is 10:1 to 1:100, advantageously 2:1 to 1:25 and especially 1:1 to 1:10.

19. A method according to claim 9, **characterized in that** compaction is achieved by the action of pressure.

20. A method according to claim 9, **characterized in that** water is allowed to act on the compacted mixture in order to dissolve out the salt.

21. A method according to claim 20, **characterized in that** the water is removed once again.

22. A method according to claim 9, **characterized in that** the compacted mixture is first stored in a CO₂ atmosphere and then the salt is dissolved out.

23. A method according to one of the preceding claims, **characterized in that** the tissue equivalents comprise tissue cells of at least two cell types, wherein the cells of the first cell type are hepatocytes and the cells of the second cell type are Langerhans islet cells.

24. A method according to claim 23, **characterized in that** the ratio of hepatocytes to Langerhans islet cells is approximately 10⁶:3000.

25. A method according to claim 23, **characterized in that** the ratio of hepatocytes to Langerhans islet cells is approximately 10⁶:3-200, advantageously approximately 10⁶:10-100, especially approximately 10⁶:20-80 and particularly preferably approximately 10⁶:35-45.

## Revendications

1. Procédé de test d'une ou plusieurs substances, dans lequel on met en culture un équivalent de tissu ; on laisse agir la ou les substances sur l'équivalent de tissu ; et on détermine si l'action de la ou des substances a provoqué une variation de l'équivalent de tissu et/ou de la ou des substances, **caractérisé en ce que** l'équivalent de tissu comprend au moins une cellule tissulaire et une matrice poreuse à base d'un polymère ou mélange de polymères biocompatibles, la matrice présentant environ 0,5% à 6% de pores ayant un diamètre moyen dans la plage de 70 à 100 µm, environ 2% à 8% de pores ayant un diamètre moyen dans la plage de 101 à 115 µm, environ 2% à 8% de pores ayant un diamètre moyen dans la plage de 116 à 130 µm, environ 1% à 7% de pores ayant un diamètre moyen dans la plage de 131 à 300 µm, environ 11% à 23% de pores ayant un diamètre moyen dans la plage de 301 à 330 µm, environ 4% à 10% de pores ayant un diamètre moyen dans la plage de 331 à 360 µm, environ 5% à 17% de pores ayant un diamètre moyen dans la plage de 361 à 390 µm, environ 7% à 19% de pores ayant un diamètre moyen dans la plage de 391 à 420 µm, environ 3% à 9% de pores ayant un diamètre moyen dans la plage de 421 à 450 µm, environ 12% à 24% de pores ayant un diamètre moyen dans la plage de 451 à 480 µm et environ 5% à 17% de pores ayant un diamètre moyen dans la plage de 481 à 510 µm, **en ce que** le degré de porosité va de 93 à 98% et **en ce que** la matrice peut être obtenue par compression d'un mélange de particules de polymère et de particules de chlorure de sodium suivie d'une élimination du chlorure de sodium par dissolution.

2. Procédé selon la revendication 1, **caractérisé en ce que** la matrice présente environ 1% à 5% de pores ayant un diamètre moyen dans la plage de 70 à 100 µm, environ 3% à 7% de pores ayant un diamètre moyen dans la plage de 101 à 115 µm, environ 3% à 7% de pores ayant un diamètre moyen dans la plage de 116 à 130 µm, environ 2% à 6% de pores ayant un diamètre moyen dans la plage de 131 à 300 µm, environ 13% à 21% de pores ayant un diamètre moyen dans la plage de 301 à 330 µm, environ 5% à 9% de pores ayant un diamètre moyen dans la plage de 331 à 360 µm, environ 7% à 15% de pores ayant un diamètre moyen dans la plage de 361 à 390 µm, 9% à 17% de pores ayant un diamètre moyen dans la plage de 391 à 420 µm, environ 4% à 8% de pores ayant un diamètre moyen dans la plage de 421 à 450 µm, environ 14% à 22% de pores ayant un diamètre moyen dans la plage de 451 à 480 µm et environ 7% à 15% de pores ayant un diamètre moyen dans la plage de 481 à 510 µm.

3. Procédé selon la revendication 1, **caractérisé en ce que** la matrice présente environ 2% à 4% de pores ayant un diamètre moyen dans la plage de 70 à 100 µm, environ 4% à 6% de pores ayant un diamètre moyen dans la plage de 101 à 115 µm, environ 4% à 6% de pores ayant un diamètre moyen dans la plage de 116 à 130 µm, environ 3% à 5% de pores ayant un diamètre moyen dans la plage de 131 à 300 µm, environ 15% à 19% de pores ayant un diamètre moyen dans la plage de 301 à 330 µm, environ 6% à 8% de pores ayant un diamètre moyen dans la plage de 331 à 360 µm, environ 9% à 13% de pores ayant un diamètre moyen dans la plage de 361 à 390 µm, environ 11% à 15% de pores ayant un diamètre moyen dans la plage de 391 à 420 µm, environ 5% à 7% de pores ayant un diamètre moyen dans la plage de 421 à 450 µm, environ 16% à 20% de pores ayant un diamètre moyen dans la plage de 451 à 480 µm et environ 9% à 13% de pores ayant un diamètre moyen dans la plage de 481 à 510 µm.

4. Procédé selon la revendication 1, **caractérisé en ce que** la matrice présente environ 3% de pores ayant un diamètre moyen dans la plage de 70 à 100 µm, environ 5% de pores ayant un diamètre moyen dans la plage de 101 à 115 µm, environ 5% de pores ayant un diamètre moyen dans la plage de 116 à 130 µm, environ 4% de pores ayant un diamètre moyen dans la plage de 131 à 300 µm, environ 17% de pores ayant un diamètre moyen dans la plage de 301 à 330 µm, environ 7% de pores ayant un diamètre moyen dans la plage de 331 à 360 µm, environ 11% de pores ayant un diamètre moyen dans la plage de 361 à 390 µm, environ 13% de pores ayant un diamètre moyen dans la plage de 391 à 420 µm, environ 6% de pores ayant un diamètre moyen dans la plage de 421 à 450 µm, environ 18% de pores ayant un diamètre moyen dans la plage de 451 à 480 µm et environ 11% de pores ayant un diamètre moyen dans la plage de 481 à 510 µm.

5. Procédé selon l'une des revendications 1 à 4, **caractérisé en ce que** le polymère biocompatible est un polymère biodégradable choisi parmi des polymères naturels tels que l'albumine, le fibrinogène, le collagène, la gélatine, la chitine, le chitosane, l'agarose, l'alginate et des polymères synthétiques tels que les polyanhydrides, la poly(ε-caprolactone) et les poly(α-hydroxyesters).

6. Procédé selon la revendication 5, **caractérisé en ce que** le polymère biodégradable est de l'acide poly(lactique-glycolique) ayant une teneur en acide lactique d'environ 85% en moles et une teneur en acide glycolique d'environ 15% en moles.

7. Procédé selon l'une des revendications 1 à 6, **caractérisé en ce que** la surface de la matrice est revêtue d'au moins une protéine de la matrice extracellulaire choisie parmi les collagènes, la laminine et la fibronectine.

8. Procédé selon la revendication 7, **caractérisé en ce que** le revêtement contient un mélange de collagène de type I, de laminine et de collagène de type IV.

9. Procédé selon l'une des revendications précédentes, **caractérisé en ce que** la matrice poreuse à base d'un polymère ou mélange de polymères biocompatibles peut être obtenue par la compression d'un mélange de particules de polymère ayant une granulométrie dans la plage d'environ 20 à 950 µm, avantageusement dans la plage d'environ 50 à 760 µm et en particulier dans la plage d'environ 108 à 250 µm, et de particules de chlorure de sodium ayant une granulométrie dans la plage d'environ 90 à 670 µm, avantageusement dans la plage d'environ 110 à 520 µm et en particulier dans la plage d'environ 250 à 425 µm, suivie de l'élimination du chlorure de sodium par dissolution.

10. Procédé selon la revendication 9, **caractérisé en ce que** le mélange de particules de chlorure de sodium se compose de 15 à 50% en poids, de préférence de 18 à 42% en poids et en particulier de 22 à 28% en poids de particules ayant une granulométrie de 250 µm à 320 µm, de 20 à 65% en poids, de préférence de 30 à 52% en poids et en particulier de 42 à 46% en poids de particules ayant une granulométrie de 330 à 380 µm, et de 15 à 62% en poids, de préférence de 25 à 42% en poids et en particulier de 29 à 33% en poids de particules ayant une granulométrie de 390 µm à 425 µm.

11. Procédé selon la revendication 9, **caractérisé en ce que** le mélange de particules de chlorure de sodium se compose de 1 à 15% en poids, de préférence de 4 à 12% en poids et en particulier de 7 à 9% en poids de particules ayant une granulométrie de 108 µm à 140 µm, de 1 à 11% en poids, de préférence de 3 à 9% en poids et en particulier de 5 à 7% en poids de particules ayant une granulométrie de 145 µm à 180 µm, de 3 à 21% en poids, de préférence de 7 à 17% en poids et en particulier de 10 à 14% en poids de particules ayant une granulométrie de 185 µm à 220 µm, de 1 à 11% en poids, de préférence de 3 à 9% en poids et en particulier de 5 à 7% en poids de particules ayant une granulométrie de 225 µm à 250 µm, de 15 à 50% en poids, de préférence de 18 à 42% en poids et en particulier de 22 à 28% en poids de particules ayant une granulométrie de 250 µm à 320 µm, de 15 à 50% en poids, de préférence de 18 à 42% en poids et en particulier de 22 à 28% en poids de particules ayant une granulométrie de 330 µm à 380 µm, et de 5 à 29% en poids, de préférence de 10 à 24% en poids et en particulier de 15 à 19% en poids de particules ayant une granulométrie de 390 µm à 425 µm.

12. Procédé selon l'une des revendications 9 à 11, **caractérisé en ce que** le mélange de particules de polymère se compose de 5 à 50% en poids, de préférence de 10 à 30% en poids et en particulier de 14 à 18% en poids de particules ayant une granulométrie de 108 µm à 140 µm, de 10 à 55% en poids, de préférence de 15 à 40% en poids et en particulier de 20 à 24% en poids de particules ayant une granulométrie de 145 µm à 180 µm, de 18 à 88% en poids, de préférence de 32 à 76% en poids et en particulier de 43 à 49% en poids de particules ayant une granulométrie de 185 µm à 220 µm, et de 5 à 45% en poids, de préférence de 10 à 28% en poids et en particulier de 14 à 18% en poids de particules ayant une granulométrie de 225 µm à 250 µm.

13. Procédé selon l'une des revendications 9 à 12, **caractérisé en ce que** le rapport en poids des particules de polymère sur les particules de chlorure de sodium se situe dans la plage de 1:100 à 1:10, avantageusement de 1:50 à 1:15 et en particulier de 1:20 à 1:18.

14. Procédé selon l'une des revendications 9 à 13, **caractérisé en ce que**, avant la compression, on ajoute une solution de polymère au mélange de particules de polymère et de particules de chlorure de sodium et on élimine le solvant.

15. Procédé selon la revendication 14, **caractérisé en ce que** le solvant dissout le polymère mais pas le sel.

16. Procédé selon la revendication 15, **caractérisé en ce que** le solvant est choisi parmi l'acétone, l'acétate d'éthyle, le chlorure de méthylène, le chloroforme, l'hexafluoro-isopropanol, des hydrocarbures aliphatiques et aromatiques chlorés et fluoré, le tétrahydrofurane, l'éthylméthylcétone, la diéthylcétone, ainsi que des mélanges de ces solvants.

17. Procédé selon la revendication 15, **caractérisé en ce que** le polymère est l'acide polyglycolique, l'acide polylactique ou l'acide poly(lactiqueglycolique) et le solvant est le chloroforme.

18. Procédé selon l'une des revendications 14 à 17, **caractérisé en ce que** le rapport en poids des particules de polymère sur le polymère dissous se situe dans la plage de 10:1 à 1:100, avantageusement de 2:1 à 1:25 et en particulier 1:1 à 1:10.

19. Procédé selon la revendication 9, **caractérisé en ce que** la compression se fait sous l'action d'une pression.

20. Procédé selon la revendication 9, **caractérisé en ce que**, pour éliminer le chlorure de sodium par dissolution, on laisse agir de l'eau sur le mélange comprimé.

21. Procédé selon la revendication 20, **caractérisé en ce que** l'on élimine à nouveau l'eau.

22. Procédé selon la revendication 9, **caractérisé en ce que** le mélange comprimé est d'abord stocké dans une atmosphère de CO₂ et ensuite le chlorure de sodium est éliminé par dissolution.

23. Procédé selon l'une des revendications précédentes, **caractérisé en ce que** l'équivalent de tissu comprend des cellules tissulaires d'au moins deux types cellulaires, les cellules du premier type étant des hépatocytes et les cellules du deuxième type des cellules d'îlots de Langerhans.

24. Procédé selon la revendication 23, **caractérisé en ce que** le rapport des hépatocytes sur les cellules d'îlots de Langerhans est d'environ 10⁶:3000.

25. Procédé selon la revendication 23, **caractérisé en ce que** le rapport des hépatocytes sur les cellules d'îlots de Langerhans se situe dans la plage d'environ 10⁶:3-200, avantageusement d'environ 10⁶:10-100, en particulier d'environ 10⁶:20-80 et de façon particulièrement préférée d'environ 10⁶:35-45.
